# EUROPEAN PATENT APPLICATION

(11) **EP 4 130 258 A1**
(43) Date of publication of application: **08.02.2023**
(21) Application number: 21189121.3
(22) Date of filing: 02.08.2021
(51) Int. Cl.: C12N 15/01, A61K 35/742

(54) **MICROORGANISMS DISPLAYING VIRAL DECOY RECEPTORS**

(71) Applicant: Evonik Operations GmbH, 45128 Essen (DE)
(72) Inventor: Berngruber, Thomas, 59069 Hamm (DE); De Groot, Raoul, 3434XC Nieuwegein (NL); Lang, Yifei, 3706AA Zeist (NL); Van Kuppefeld, Frank, 5345TC Oss (NL); Flügel, Monika, 33803 Steinhagen (DE); Molck, Stella, 33602 Bielefeld (DE)
(74) Representative: Evonik Patent Association

(57) **Abstract**

The application relates to the identification and application of microorganisms which inhibit virus binding to viral target receptors by displaying decoy receptors on their surface, conferring competitive binding affinity to the viral particles.

## Description

The application relates to the identification and application of microorganisms which inhibit virus binding to viral target receptors by displaying decoy receptors on their surface, conferring competitive binding affinity to the viral particles.

Many viruses use glycan-protein interactions for attachment to their host cells as the first step in the infection cycle. Thus, the application of purified glycans has been disclosed for the prevention, amelioration or cure of viral infections as such purified glycans can act as viral decoy-receptors and by that help to prevent viral infections by competitive binding to the receptor binding molecule of the viral particle (Van Breedam et al. (2014), FEMS Microbiology Reviews, 38(4), 598-632; Thompson et al. (2019), Current Opinion in Virology, 34, 117-129; Brogden, K. A. (2005), Nat Rev Microbiol, 3(11), 238-250; Boas et al. (2019), Cellular and Molecular Life Science, 18, 3525-3542; US5589453; Al Kassaa et al. (2014), Probiotics & Antimicro. Prot. 6:177-185; Starosila et al. (2017), Antimicrob Agents Chemother. 61(7) e00539-17).

Substances which have been disclosed as viral decoy-receptors up to now are usually either purified from animal products (e.g. human milk oligosaccharides ("HMOs") from milk, eggs and heparin or chondroitin from cartilage or chitin/chitosan from shells of crustaceans), purified from slow-growing macroalgae (e.g. carrageen from *Eucheuma* species) or produced by fermentation or expression in genetically modified organisms (GMO).

WO 2009/027057 discloses the use of sulfated polysaccharides for the preparation of anti-viral compositions. Han et al. (2012, Biotech. Adv., 30(6), 1268-1278) disclose a method for producing HMOs biotechnologically. WO 2017/221208 discloses the use of compositions comprising HMOs for the treatment of viral or bacterial infections. EP2658399 discloses compositions comprising HMOs for treating enteric rotavirus infections. Koketsu et al. (1995, Journal of Agricultural and Food Chemistry, 43(4), 858-861) disclose the use of sialyloligosaccharides from egg yolk as an inhibitor of rotaviral infection. WO 2019/162425 discloses a method of preparing glycan compositions. Azagra-Boronat et al. (2018; Frontiers in Cellular and Infection Microbiology, 8: 372) disclose that supplementation with 2'-FL and scGOS/IcFOS ameliorates rotavirus-induced diarrhea in suckling rats. Li et al. (2014; ISME Journal, 8(8), 1609-1620) disclose that HMOs shorten rotavirus-induced diarrhea and modulate piglet mucosal immunity and colonic microbiota.

US 8795651 B2 describes strains of *Lactobacillus sakei* and *Lactobacillus plantarum* which can produce sialic acid and can be applied as live cells in food. As a purpose of the sialic acid enriched food is mentioned a positive influence on the cognitive development, to boost immunity and/or to improve gut function. There is no indication that the produced sialic acid could function as viral decoy-receptor or could be used for the treatment of viral infections.

US 5733540 proposes the construction of genetically modified microorganisms with virus-binding activities, by heterologous expression of virus receptor proteins which are displayed on the microbial cell surface. In addition, this disclosure proposes to genetically engineer sialyl-transferase into microorganisms to produce sialic acid glycan decoy receptors but does not demonstrate feasibility of this idea. Notwithstanding, such genetic modifications will create a GMO, which is not acceptable for food stuff or animal feed by certain regulatory bodies and can be non-desirable for consumers of the food stuff or animal growers.

EP 2016081308W describes a mixture of several purified viral decoy-receptor glycans (LNnT, LNT, 2'-FL, 3'-SL, 6'-SL and DFL) to arrive at a product which covers a large spectrum of viruses. Importantly, each of these glycans needs to be produced in purified form before the mixing - this procedure significantly increases production costs.

WO 2019/162425 describes the purification of sialylated compounds from animal gastric mucin, predominantly from swine. Such products of animal origin are however undesired for certain applications and in certain cultural backgrounds (feed/food, kosher, halaal). Furthermore, this disclosure does not mention the application of such glycans in the prevention or amelioration of viral infections.

The presence of specific glycans on the surface of microorganisms has been demonstrated in two key publications using plant-lectins. Gao et al. (2010; Analytical Chemistry, 82(22), 9240-9247) demonstrate the binding of various microorganisms to plant lectins, which indicates the presence of specific glycans on the surface of these microorganisms. The publication does not demonstrate the binding of viral lectins to these glycans and does not mention the potential application of such microorganisms in the prevention or amelioration of viral infections. Yasuda et al. (2011; Applied and Environmental Microbiology, 77(13), 4539-4546), uses a lectin microarray to identify the glycans produced by several *Lactobacillus casei* strains. This publication does, however, not demonstrate the binding of viral receptor-binding proteins by these glycans and does not mention the potential application of such microbes as anti-viral agents.

Tong et al. (Cell Microbiol. 2018 Apr;20(4)) demonstrates that influenza virus can adhere to sialic acid glycan residues on the surface of the bacterial pathogen *Streptococcus suis.* Since *Streptococcus suis* is a well-known pathogen, its application in feed and food was obviously neither considered nor discussed. Pathogens like *Streptococcus suis* cannot be used in feed or food applications.

Wang et al. (2013; PLoS ONE 8(1): e53043) and Kreuzer et al. (Veterinary Research 2012 43:58) did show that the strain *Enterococcus faecium* NCIMB 10415 can bind Influenza virus and transmissible gastroenteritis virus onto its cell surface and reduce the shedding of other viruses in weaning piglets. But these publications did not show that viral decoy receptors would be responsible for the observed effects.

The known sources for viral decoy receptors as described before have several disadvantages which limit significantly their scope of application. Thus, products of animal origin are not acceptable for certain applications and cultural backgrounds; macroalgae are slow growing and require photoreactors or open pond systems; and production of virus-receptor decoy-glycans in GMOs requires purification to eliminate living cells and recombinant biomass for regions where GMOs are not accepted as food or feed by consumers or by a regulatory body.

The disadvantages and limitations of the state of the art are overcome by the subject of the present application. According to the invention it was surprisingly found out that some naturally occurring microorgansims are already able to produce viral decoy receptors in sufficient amounts, so that they can be used as viral decoy receptors without the need of genetic modification.

Further, endospore-forming microorganisms, in particular of the genus *Bacillus,* have been identified as particularly suitable viral decoy receptor producing microorganisms.

Such endospore-forming microorganisms of the genus *Bacillus* are particularly well suited for application in animal feed, water or rearing environment due to the good stability and shelf-life of *Bacillus* endospores during the various steps of application in animal husbandry or aquaculture.

The viral decoy receptor producing microorganisms according to the invention can be identified by a screening method, wherein viral particles or their receptor-binding moieties are immobilized on a carrier, in particular on a surface or on a (nano)particle.

Thus, a first subject of the present invention are microorganisms which display viral decoy-receptors on their surface and preparations thereof.

Thus, a further subject of the present invention are also endospore-forming microorganisms, in particular of the genus *Bacillus,* which display viral decoy receptors on their surface and preparations thereof.

Thus, a further subject of the present invention is also a method of identifying viral decoy receptor producing microorganisms, wherein viral particles or their receptor-binding moieties are immobilized on a carrier and microorganisms are subsequently analyzed for their ability to bind to the viral particles or receptor-binding moieties on the carrier. The method is preferably carried out basically as disclosed by Li et al. (2017; PNAS 114 (40), E8508-8517). The main difference is that microorganisms are used in the screening. Further, different kinds of viral receptors were displayed in the assay to check the microorganisms for their ability to bind to such viral receptors.

The microorganisms of the invention are preferably natural, non-genetically engineered (non-GMO), viral decoy-receptor producing microorganisms ("NVDRPMs"). The NVDRPMs may in particular be identified from a collection of microorganisms. The microorganisms of the invention, in particular the non-GMO microorganisms, may also be mutants of such naturally occurring microorganisms.

Principally, according to the invention the term mutant refers to any kind of mutant obtained from a parent strain, i.e. also to mutants which are obtained by genetically modifying the genetic material of the strain (i.e. also to GMOs). But the microorganisms according to the invention are preferably not genetically modified, i.e. non-GMO. This means that the microorganisms are preferably either naturally occurring microorganisms or spontaneous mutants of such naturally occurring microorganisms. The terms "spontaneous mutant" and "non-GMO mutant" refers to mutants that arise from naturally occurring microorganisms without genetically modifying the microorganisms by applying gene technological and/or biotechnological methods. Such spontaneous mutants may be obtained by classical methods of natural selection, such as growing the microorganisms in the presence of UV light and/or by applying high temperature or protoplast formation and/or in the presence of a certain antibiotic to which the parent strain is susceptible. Spontaneous mutants might be further, but less preferably, obtained by using mutagens, i.e. chemical substances which induce the formation of mutants. As formation of spontaneous mutants by using mutagens is less preferred, in a preferred embodiment of the invention the spontaneous mutants and/or non-GMO mutants are obtained without the use of such mutagens.

One particular embodiment of the invention are naturally non-occurring mutants, in particular spontaneous mutants as defined before, of the microorganisms of the invention. The mutants according to the invention, in particular the spontaneous mutants, are preferably also able to bind viruses and thus possess also viral decoy receptors, more preferably the mutants have at least the same capacity to bind viruses like the naturally occurring parent strains, from which they are derived from. The mutants have furthermore preferably the same further identifying characteristics like the parent strain from which they were derived.

Mutants of the microorganisms can easily be tested for their biological activity, in particular improved biological activity, in particular with respect to their ability to bind and/or inactivate viral particles, or for their ability to enhance one or more of the indicia of human or animal health, in particular gut health.

The "mutants" or "variants" of the invention, in particular the spontaneous mutants, if not explicitly described otherwise, preferably have a sequence identity of at least 95, 96 or 97 %, more preferably of at least 98, 99 or 99.5 %, in particular of at least 99.8, 99.9 or 99.95 %, to the genomic DNA of the parent strain. I.e. in this context the term "sequence identity" always relates to the complete genomic DNA, if not explicitly mentioned otherwise.

The microorganisms according to the invention are preferably further non-pathogenic, in particular non-pathogenic to human beings, animals and preferably also non-pathogenic to plants.

The microorganisms according to the invention are preferably selected from a list of microorganisms generally recognized as safe ("GRAS") or listed as direct fed microbials ("DFM") or probiotics as stated in relevant listings, as for example, but not limited to the EFSA QPS list, the AAFCO list or the list by the Chinese Ministery of Agriculture (MOA).

The microorganisms according to the invention have preferably sensitivity for at least five antibiotics, more preferably for at least six, eight or ten antibiotics, above all for at least 11 or 12 antibiotics, wherein the antibiotics are preferably selected from tylosin, nalidixic acid, trimethoprim, apramycin, sulfonamide, ampicillin, vancomycin, gentamicin, kanamycin, streptomycin, erythromycin, clindamycin, tetracycline, chloramphenicol, nourseothricin, daptomycin and virginiamycin. In a very preferred embodiment the microorganisms according to the invention have sensitivity to all antibiotics as mentioned before.

To be "sensitive for antibiotics" means that growth of the microorganism is inhibited by the antibiotic under conditions where the microorganism would otherwise grow. Sensitivity for antibiotics is preferably tested in accordance with the CLSI guidelines (M07-A8 and M45-A2). A Bacillus strain is preferably considered sensitive, if growth is only detected at or below the breakpoint concentration specified in EFSA Journal 2012; 10(6):2740 for vancomycin, gentamicin, kanamycin, streptomycin, erythromycin, clindamycin, tetracycline and chloramphenicol. Concerning tylosin, nalidixic acid, trimethoprim, apramycin, sulfonamide, ampicillin, nourseothricin, daptomycin and virginiamycin, for which no breakpoint is given by EFSA for Bacillus, the breakpoint is preferably 4 mg/L.

The microorganisms of the invention are preferably selected from endospore-forming bacteria, in particular from the genus Bacillus and are more preferably selected from the species *B*. *subtilis*, *B. amyloliquefaciens, B. velezensis, B. licheniformis, B. paralicheniformis, B. pumilus, B. megaterium, B. lentus, B. laterosporus, B. alevi, B. cereus, B. badius, B. thurigiensis, B. coagulans, B. siamensis, B. glycinifermentans, B. methylotrophicus, B. thuringensis, B. polyfermenticus, B. vallismortis, B. tequilensis, B. atrophaeus, B. mojavensis, B. sonorensis, B. inaquosus* and *B*. *safensis*, most preferably they are of the species *Bacillus subtilis, Bacillus velezensis, Bacillus amyloliquefaciens, Bacillus pumilus* or *Bacillus megaterium,* above all they are of the species *Bacillus subtilis* or *Bacillus velezensis.*

In the way of a screening of about 200 naturally occurring Bacillus strains, 27 were identified to be able to bind to virolectins of a coronavirus, in particular to PEDV particles. 16 of those 27 strains were able to bind in addition to virolectins of a rotavirus, in particular to VP8*. The identified binding strains were of the species *Bacillus subtilis, Bacillus velezensis, Bacillus pumilus* and *Bacillus megaterium*. Of the identified strains, four were selected, because they exhibited some further positive characteristics which identified them as particularly suitable for feed applications. The selected strains were deposited with the DSMZ (Deutsche Sammlung für Mikroorgansimen und Zellkulturen, Inhoffenstraße 7B, 38124 Braunschweig, Germany) on April 23, 2021 under the provisions of the Budapest Treaty on the International Recognition of the Deposit of Microorganisms for the Purpose of Patent Procedure and obtained the following deposit numbers: DSM 33855, DSM 33856, DSM 33857 and DSM 33858. Of the four deposited strains, three are of the species *B*. *subtilis* (DSM 33855, DSM 33856 and DSM 33858), while one belongs to the species *B*. *velezensis* (DSM 33857).

Thus, in a very preferred embodiment of the invention, the microorganisms of the invention are selected from the strains DSM 33855, DSM 33856, DSM 33857 and DSM 33858 and variants thereof as well as from mixtures of these four strains and variants thereof.

Preferably the strain DSM 33855 and variants thereof have the following characteristics:
a) a 16S rDNA sequence with a sequence identity of at least 99.5%, preferably at least 99.8%, more preferably 100%, to the sequence according to SEQ ID NO: 1;
b) a yqfD sequence with a sequence identity of at least 99.5%, preferably at least 99.8%, more preferably 100%, to the sequence according to SEQ ID NO: 2;
c) a gyrB sequence with a sequence identity of at least 99.5%, preferably at least 99.8%, more preferably 100%, to the sequence according to SEQ ID NO: 3;
d) an rpoB sequence with a sequence identity of at least 99.5%, preferably at least 99.8%, more preferably 100%, to the sequence according to SEQ ID NO: 4;
e) a groEL sequence with a sequence identity of at least 99.5%, preferably at least 99.8%, more preferably 100%, to the sequence according to SEQ ID NO: 5.

Preferably the strain DSM 33856 and variants thereof have the following characteristics:
a) a 16S rDNA sequence with a sequence identity of at least 99.5%, preferably at least 99.8%, more preferably 100%, to the sequence according to SEQ ID NO: 6;
b) a yqfD sequence with a sequence identity of at least 99.5%, preferably at least 99.8%, more preferably 100%, to the sequence according to SEQ ID NO: 7;
c) a gyrB sequence with a sequence identity of at least 99.5%, preferably at least 99.8%, more preferably 100%, to the sequence according to SEQ ID NO: 8;
d) an rpoB sequence with a sequence identity of at least 99.5%, preferably at least 99.8%, more preferably 100%, to the sequence according to SEQ ID NO: 9;
e) a groEL sequence with a sequence identity of at least 99.5%, preferably at least 99.8%, more preferably 100%, to the sequence according to SEQ ID NO: 10.

Preferably the strain DSM 33857 and variants thereof have the following characteristics:
a) a 16S rDNA sequence with a sequence identity of at least 99.5%, preferably at least 99.8%, more preferably 100%, to the sequence according to SEQ ID NO: 11;
b) a yqfD sequence with a sequence identity of at least 99.5%, preferably at least 99.8%, more preferably 100%, to the sequence according to SEQ ID NO: 12;
c) a gyrB sequence with a sequence identity of at least 99.5%, preferably at least 99.8%, more preferably 100%, to the sequence according to SEQ ID NO: 13;
d) an rpoB sequence with a sequence identity of at least 99.5%, preferably at least 99.8%, more preferably 100%, to the sequence according to SEQ ID NO: 14;
e) a groEL sequence with a sequence identity of at least 99.5%, preferably at least 99.8%, more preferably 100%, to the sequence according to SEQ ID NO: 15.

Preferably the strain DSM 33858 and variants thereof have the following characteristics:
a) a 16S rDNA sequence with a sequence identity of at least 99.5%, preferably at least 99.8%, more preferably 100%, to the sequence according to SEQ ID NO: 16;
b) a yqfD sequence with a sequence identity of at least 99.5%, preferably at least 99.8%, more preferably 100%, to the sequence according to SEQ ID NO: 17;
c) a gyrB sequence with a sequence identity of at least 99.5%, preferably at least 99.8%, more preferably 100%, to the sequence according to SEQ ID NO: 18;
d) an rpoB sequence with a sequence identity of at least 99.5%, preferably at least 99.8%, more preferably 100%, to the sequence according to SEQ ID NO: 19;
e) a groEL sequence with a sequence identity of at least 99.5%, preferably at least 99.8%, more preferably 100%, to the sequence according to SEQ ID NO: 20.

The viral decoy receptors which are produced and/or displayed by the microorganisms are preferably glycotopes, i.e. glycans or a glycan comprising structure, which contain at least one sugar unit, preferably from one to ten sugar units. The sugar unit may in particular be a sugar alcohol, a sugar acid or a sugar amine, wherein the sugar alcohol is preferably lactose, the sugar acid is preferably sialic acid (Sia) and the sugar amine is preferably selected from N-acetylgalactosamine, N-acetylglucosamine and N-acetylmannosamine.

The viral decoy receptors as produced and/or displayed by the microorganisms are preferably identical to naturally occurring binding sites of viruses. In particular, the viral decoy receptors are preferably identical to glycotopes, i.e. glycans or glycan containing structures, which serve for the binding of viruses in human beings, animals or plants.

In a preferred embodiment, the viral decoy receptor is or comprises a glycan, preferably selected from sialic acid (Sia) and Sia containing glycans, in particular selected from NeuGc alpha 2,3 Sialic acids, Neu5,9Ac2 Sialic acids, α2,3 N-linked and O-linked sialic acids, NeuAc alpha 2,6 Sialic acids, alpha 2,3 Sialic acids, alpha 2,6 Sialic acids, 9-O-acetylated Sialic acids, Neu5Gc Sialic acids, Neu5Ac Sialic acids and linear sialylated pentasaccharides, glycosaminoglycans, mucin-core-2, LNT (lacto-N-tetraose), LNnT (lacto-N-neotetraose), heparan sulfate, chondroitin sulfate and A/O Histo-blood group antigen (HBGA).

In a very preferred embodiment of the invention, the glycan is selected from sialic acid (Sia), a Sia containing glycan, mucin-core-2, LnNT (lacto-N-neotetraose) and LnT (lacto-N-tetraose).

The virolectin which is bound by the viral decoy receptors is preferably a virolectin of a coronavirus, preferably a variant of the porcine epidemic diarrhea virus major spike protein PEDV, in particular PEDV-S1, or a virolectin of a rotavirus, preferably of a group A rotavirus, more preferably a variant of the rotavirus receptor binding protein VP8*, in particular the variant P6_Z84_VP8* or P19_VP8*.

PEDV (porcine epidemic diarrhea virus) is a coronavirus. Coronaviruses are enveloped, positive-strand RNA viruses which are generally associated with respiratory and/or enteric infection of a large variety of mammals and birds. PEDV, in particular PEDV-S1, is known to bind to sialic acid (Sia) and/or to Sia containing glycans, in particular to α2,3-linked Sia.

Group A rotaviruses are naked RNA viruses with a double strand eleven-segmented genome and a leading cause of severe dehydrating diarrhea in humans and animals. There are six viral proteins (VPs) which form the virus particle of rotaviruses, wherein the VP4 spikes mediate attachment and entry into the cell. VP4 is activated through proteolytic cleavage as an essential step for particles to become infectious. Cleavage of VP4 leads to the C-terminal subunit VP5 and the N-terminal VP8*, which is a lectin domain that allows viral attachment to the host cells. The VP8* lectins of P[6] and P[19] have been reported to be able to bind to mucin core-2 (GlcNAcβ1-6(Galβ1-3)GalNAc) and type I HBGA precursors with a Galβ1-3GlcNAc motif (LNT), with GlcNAc as sole common denominator and central binding residue.

It is known that sialic acid, in particular sialic acid linked to glycoproteins and gangliosides, is used by many viruses as a receptor for cell entry. It is generally assumed that viruses which are able to bind to α2,3-linked Sia are generally able to bind to Sia in structures like NeuGc alpha 2,3 Sialic acids, Neu5,9Ac2 Sialic acids, α2,3 N-linked and O-linked sialic acids, NeuAc alpha 2,6 Sialic acids, alpha 2,3 Sialic acids, alpha 2,6 Sialic acids, 9-O-acetylated Sialic acids, Neu5Gc Sialic acids, Neu5Ac Sialic acids and linear sialylated pentasaccharides The microorganisms according to the invention preferably display as viral decoy receptors at least one, in particular at least two, Sia containing glycans as mentioned before. Viruses which are known to bind to such structures include important human and animal pathogens from all viral classes, such as Influenza virus, including Influenza A, B and C and Parainfluenza viruses, Mumps virus, Corona virus, Norovirus, Rotavirus, and DNA tumor viruses, Isavirus, Torovirus, Enteroviruses, Carbovirus, Rubulavirus, Respirovirus, Avulavirus, Bocavirus, Protoparvovirus and Polyomavirus.

Thus, the viruses which are bound by the viral decoy receptors according to the invention, in particular to a sialic acid containing structure of the viral decoy receptors, preferably to a structure as mentioned before, are preferably selected from the families Myxoviridae, Coronaviridae, Caliciviridae, Reoviridae, Noroviridae, Picornaviridae, Adenoviridae, Anelloviridae, Asfarviridae, Baculoviridae, Circoviridae, Geminiviridae, Hepadnaviridae, Iridoviridae, Nanoviridae, Nimaviridae, Nudiviridae, Papillomaviridae, Parvoviridae, Polyomaviridae, Flaviviridae, Bunyaviridae, Filoviridae, Arenaviridae and Poxviridae.

In a preferred embodiment of the invention the virus is selected from Myxoviridae, in particular from Avian Influenza A virus, BK virus, Human Influenza A virus, Human parainfluenza 1, Human parainfluenza 3, Newcastle disease virus, Influenza B virus, Influenza C virus, Bovine Influenza D virus, Infectious Salmon Anemia virus (ISAV), Tilapia Lake virus (TiLV), Sendai virus, Rubulavirus, Respirovirus and Avulavirus.

In a further preferred embodiment of the invention the virus is selected from Reoviridae, in particular from Bluetongue virus, Orbivirus, Reovirus, Rotavirus, in particular Porcine Rotavirus or Rhesus Rotavirus.

In a further preferred embodiment of the invention the virus is selected from Coronaviridae, in particular from Alphacoronaviruses, Betacoronaviruses, Gammacoronaviruses, Deltacoronaviruses, Toroviruses, in particular bovine coronaviruses like TGEV, SECoV, PEDV or PDCoV, preferably PEDV, or human coronavirus OC43 (HCoV-OC43).

In a further preferred embodiment of the invention the virus is selected from Picornaviridae, in particular from Carbovirus, Enteroviruses, in particular Enterovirus 70, Enterovirus 71 or Enterovirus D68 (Rhinovirus 87), Coxsackievirus A24 (CVA24v).

In a further preferred embodiment of the invention the virus is selected from Adenoviridae and is preferably Human Adenovirus 37 (HAdV-37).

In a further preferred embodiment of the invention the virus is selected from Polyomaviridae and is preferably Mouse polyomavirus mPy,

In a further preferred embodiment of the invention the virus is selected from Polyomaviridae and is preferably Polyomavirus.

In a further preferred embodiment of the invention the virus is selected from Parvoviridae, in particular from Bocavirus, Protoparvovirus, Bovine parvovirus and Minute virus of mice.

Thus, a further embodiment of the present invention is a pharmaceutical composition, containing at least one microorganism and/or at least one preparation according to the invention, in particular a pharmaceutical composition for treating and/or preventing and/or mitigating the course of a viral disease as caused by a virus as mentioned before.

Thus, a further embodiment of the present invention is a method of treating and/or preventing and/or mitigating the course of a disease, in particular of a viral disease as caused by a virus as mentioned before, wherein a microorganism and/or a preparation and/or a pharmaceutical composition according to the invention is administered to an animal or human being in need thereof.

Thus, a further embodiment of the present invention is also the use of at least one microorganism according to the invention for preparing a feedstuff, a foodstuff or a pharmaceutical composition, in particular a pharmaceutical composition for treating and/or preventing and/or mitigating the course of a disease, in particular of a viral disease as caused by a virus as mentioned before.

Thus, a further embodiment of the present invention is also a microorganism and/or a preparation and/or a composition, in particular a pharmaceutical composition, according to the invention, for treating and/or preventing and/or mitigating the course of a disease, in particular a viral disease as caused by a virus as mentioned before.

The disease, in particular the viral disease, which is treated and/or prevented and/or mitigated by the microorganisms according to the invention is very preferably diarrhea, in particular porcine or avian diarrhea, preferably as caused by a coronavirus, in particular by porcine epidemic diarrhea virus (PEDV), or as caused by a rotavirus, in particular a group A rotavirus.

Thus, a further embodiment of the present invention is also the use of a microorganism and/or a preparation and/or a composition according to the invention for feeding animals.

Thus, a further embodiment of the present invention are also feed and food compositions containing at least one microorganism according to the invention.

Thus, a further embodiment of the present invention is also a method of feeding animals, wherein at least one microorganism and/or at least one preparation and/or at least one composition according to the invention is administered to animals.

Thus, a further embodiment of the present invention is also the use of a microorganism and/or a preparation and/or a composition according to the invention for reducing the amount of free viruses in a feed or food composition, before the feed or food composition is administered to animals.

By administering a microorganism to animals the free amount of pathogenic viruses, preferably the amount of pathogenic Coronaviruses or Rotaviruses, in particular the amount of porcine epidemic diarrhea virus, at the site of application, in particular in an animal gastrointestinal tract or in an oral or nasopharyngeal cavity, preferably in an animal gut, is preferably reduced by at least 10%, preferably by at least 20, 30 or 50%.

Very preferably the microorganisms according to the invention produce and/or display at least two different kinds, in particular exactly two different kinds, preferably at least three or four different kinds, in particular exactly three or four different kinds, of viral decoy receptors, wherein the viral decoy receptors are preferably selected from naturally occurring glycans, in particular from glycans such as Sia and Sia containing glycotopes, mucin-core-2, LNnT and LNT and/or from further glycans which are able to bind to viral lectins, in particular to PEDV-S1 and rotavirus VP8* variants.

Very preferably, the microorganisms according to the invention are able to bind at least two, in particular exactly two, more preferably at least three or four, in particular exactly three or four, different kinds of viruses, i.e. they display at least two, three or four different kinds of glycans or glycotopes which are able to bind to virolectins of different kinds of viruses and/or to different kinds of glycans or glycotopes which may also be located on the same kind of virus.

In a preferred embodiment of the invention, the microorganisms according to the invention display as viral decoy receptors at least one Sia containing glycan and at least one non-Sia containing glycan, wherein the Sia containing glycan is preferably selected from Sia, NeuGc alpha 2,3 Sialic acids, Neu5,9Ac2 Sialic acids, α2,3 N-linked and O-linked sialic acids, NeuAc alpha 2,6 Sialic acids, alpha 2,3 Sialic acids, alpha 2,6 Sialic acids, 9-O-acetylated Sialic acids, Neu5Gc Sialic acids, Neu5Ac Sialic acids and linear sialylated pentasaccharides, and the non-Sia containing glycan is preferably selected from lactose containing glycans and N-acetylated sugar amine containing glycans, more preferably from glycosaminoglycans, mucin-core-2, LNT (lacto-N-tetraose), LNnT (lacto-N-neotetraose), heparan sulfate, chondroitin sulfate and A/O Histo-blood group antigen (HBGA).

The microorganisms according to the invention preferably display at least 5, preferably at least 10 or 20, more preferably at least 40 or 60, above all at least 80 or 100, glycan binding sites on the surface. Accordingly, the microorganisms according to the invention are preferably able to bind at least 5, preferably at least 10 or 20, more preferably at least 40 or 60, above all at least 80 or 100, different virus particles on their surface. Preferred ranges are 5 to 200, 5 to 100, 20 to 200 and 20 to 100 glycan binding sites.

In case that the microorganisms display more than one kind of viral decoy receptor on the surface, then the microorganisms preferably display at least one of those different kinds of viral decoy receptors in an amount of at least 5, 10 or 20, preferably in an amount of at least 30 or 50 units on the surface, but very preferably they display at least two or three, in particular each of the viral decoy receptors as present, in an amount of at least 5, 10 or 20, more preferably in an amount of at least 30 or 50 units on the surface. Correspondingly, the microorganisms according to the invention are preferably able to bind at least two, in particular two or three, more preferably at least three, in particular three, four or five, different viral particles with affinity to different binding sites, in an amount of at least 5, 10 or 20 units each, preferably in an amount of at least 30 or 50 units each.

The microorganisms according to the invention are preferably further able to produce at least one substance which impairs the stability or functionality of viral particles and/or inactivate viral particles, wherein the substances are preferably selected from proteases and biosurfactants. Binding on the microbial surface brings the viral particle into the proximity of the NVDRPM where the concentration of the virus inactivating substances is highest and the virucidal effect is most effective.

In a very preferred embodiment of the invention, the microorganisms according to the invention additionally possess probiotic activity and/or can be used as direct fed microbials.

The microorganisms according to the invention in particular preferably possess at least one, more preferably at least two, three, four or five, further characteristics selected from:
a) Ability to grow fast in large-scale bioreactors;
b) Ability to multiply and produce viral decoy receptors *in situ*;
c) Ability to grow in the presence of bile;
d) Ability to grow in the presence of formic acid, acetic acid, propionic acid and/or lactic acid;
e) Ability to grow under high salt conditions;
f) Ability to form endospores;
g) Ability to grow anaerobically;
h) Ability to inhibit pathogenic bacteria;
i) Ability to produce at least one enzyme, preferably selected from amylase, lipase, catalase, cellulase, xylanase and protease;
j) Sensitivity with respect to selected antibiotics;
k) Ability to produce biosurfactants, in particular surfactin.

The microorganisms of the invention, in particular microorganisms of the genus Bacillus, are preferably able to grow fast in large-scale bioreactors. This means in particular heterotrophic growth in the absence of light with doubling rates lower than 60 minutes within stirred tank reactors.

The microorganisms of the invention, in particular microorganisms of the genus Bacillus, are further preferably able to multiply and produce viral decoy receptors *in situ*. This ability can establish a long-lasting effect with lower dosing compared to non-multiplying viral decoy receptors like glycan substances as currently used.

The microorganisms according to the invention, in particular microorganisms of the genus Bacillus, are preferably able to grow in the presence of bile, in particular in the presence of 0.3 wt.-% porcine bile. More preferably, they are able to proliferate fast under such high bile concentrations. In particular they are preferably characterized by an AUC5 performance value of at least 0.5, preferably at least 0.8, in particular at least 1.0, above all at least 1.2, and/or by an AUC10 performance value of at least 3.0, preferably at least 4.0, above all at least 5.0, in presence of 0,3 wt.-% porcine bile, respectively.

The microorganisms according to the invention, in particular microorganisms of the genus Bacillus, are preferably able to grow in the presence of formic acid, acetic acid, propionic acid and/or lactic acid, in particular at pH 6 and/or in the presence of 0.05 wt.-% formic acid, 0.05 wt.-% acetic acid, 0.05 wt.-% propionic acid and/or 0.2 wt.-% lactic acid.

The microorganisms according to the invention, in particular microorganisms of the genus Bacillus, are preferably able to grow under high salt conditions, in particular in the presence of 1 wt.-% of NaCl, more preferably in the presence of 2, 3 or 5 wt.-% NaCl, in particular at pH 7, for at least 24 hours.

The microorganisms according to the invention are preferably further characterized by exhibiting at least one or two, preferably all, of the following enzymatic activities: cellulase activity; xylanase activity; protease activity.

The microorganisms according to the invention are preferably able to grow under anaerobic conditions. They are preferably further able to produce lactate under anaerobic conditions. Further, they preferably may be able to degrade water-insoluble cellulose and/or protein under such anaerobic conditions.

Besides their ability to bind pathogenic viruses, the microorganisms according to the invention, in particular microorganisms of the genus Bacillus, are preferably also able to inhibit and/or decrease the growth of at least one pathogenic bacterium, in particular in a human or animal gut, wherein the pathogenic bacterium is preferably selected from *Clostridia*, *Listeria*, *Salmonella*, *Enterococci*, *Staphylococci, Aeromonas, Streptococci, Campylobacter, Escherichia coli, Shigella, Haemophilus, Brachyspira, Flavobacterium, Serratia, Yersinia, Edwardsiella, Rennibacterium, Pasteurella* and *Vibrio.*

Thus, the microorganisms, preparations and compositions of the invention are preferably used to inhibit or decrease the growth of pathogenic bacteria, in particular in an animal gut, on an animal skin or in the gills of aquatic animals, and/or they are preferably administered or fed to an animal in an amount effective to inhibit and/or decrease the growth of pathogenic bacteria, wherein the pathogenic bacteria are preferably selected from *Clostridia*, in particular from *C*. *perfringens*, *C. difficile*, *C. novyi*, *C. septicum* and C. *colinum,* from *Listeria,* in particular from *L. monocytogenes, L. seeligeri* and L. *welshimeri,* from *Salmonella,* in particular *S*. *enterica* including the subspecies *enterica, arizonae, bongori* and in particular the serovars, S. *gallinarum, S. pullorum, S. typhimurium, S. enteritidis, S. cholerasuis, S. heidelberg* and S. *infantis,* from *Enterococci,* in particular E. *faecalis, E. faecium* and *E. cecorum,* from *Staphylococci,* in particular S. *aureus,* from *Aeromonas,* in particular from A. *hydrophila* and A. *salmonocida,* from *Streptococci,* in particular S. *suis, S. gallinaceus* and S. *agalactiae,* from *Campylobacter,* in particular C. *jejuni* and C. *coli,* from *Escherichia coli,* from *Haemophilus,* in particular *Haemophilus parasuis,* from *Brachyspira, in particular Brachyspira hyodysenteriae,* from *Flavobacterium,* in particular *Flavobacterium columnare,* from *Serratia,* in particular S. *liquefaciens,* from *Yersinia,* in particular *Y. ruckeri,* from *Edwardsiella,* in particular E. *tarda* and E. *ictaluria,* from *Rennibacterium,* in particular R. *salmoninarum,* from *Pasteurella,* in particular P. *piscicida,* and from *Vibrio,* in particular *V. parahemolyticus, V. harveyi, V. anguillarum, V. ordalii, V. alginolyticus, V. fischeri* and *V. salmonicida.*

Thus, a further subject of the invention are also the microorganisms, preparations and compositions of the invention themselves for inhibiting and/or decreasing the growth of pathogenic bacteria in an animal gut or on an animal skin or in the gills of aquatic animals, and/or for preventing or curing the diseases connected with said pathogenic bacteria, wherein the pathogenic bacteria are preferably selected from the bacteria as mentioned in the paragraph above. By decreasing the amount of pathogenic bacteria, the compositions of the present invention additionally contribute to maintain an overall healthy microflora, in particular in the gut or on the skin of animals or in the gills of aquatic animals.

In a preferred embodiment of the invention, the microorganisms of the invention, in particular the strains DSM 33855, DSM 33856, DSM 33857 and DSM 33858 and variants thereof, are able to inhibit or decrease the growth of at least one pathogen, in particular at least two pathogens, preferably all pathogens, selected from *Clostridium perfringens, Streptococcus suis* and *Enterococcus cecorum.*

The occurrence and/or increased growth of *Clostridium perfringens* can lead to the outbreak of gut diseases, in particular to the outbreak of necrotic enteritis in swine and poultry. It can also lead to the outbreak of further diseases like bacterial enteritis, gangrenous dermatitis and cholangiohepatitis. Even the mildest form of infection by C. *perfringens* can already be accompanied by diarrhea, which results in wet litter and by that may lead to secondary diseases like foot pad dermatitis. While C. *perfringens* type C generally is considered to be the primary cause of necrotic enteritis and necrohemorrhagic enteritis in piglets, type A has been linked to enteric disease in suckling and feeding pigs with mild necrotic enterocolitis and villous atrophy.

*S*. *suis* is an important pathogen in pigs and one of the most important causes of bacterial mortality in piglets after weaning causing septicemia, meningitis and many other infections.

*E. cecorum* is known to cause lameness, arthritis and osteomyelitis in broilers usually caused by an inflammation of a joint and/or bone tissue. Further *E*. *cecorum* can cause an inflammation of the pericardium.

Furthermore, some microorganisms of the invention are preferably able to inhibit or decrease the growth of at least one further pathogen, selected from *E*. *coli, Staphylococcus aureus* subsp. aureus and *Vibrio parahaemolyticus.*

In particular the strains DSM 33855, DSM 33856 and DSM 33857 and variants thereof, are preferably able to inhibit the growth of pathogenic *Salmonella enterica* subsp. enterica strains and/or pathogenic *E. coli* strains.

Further, in particular the strains DSM 33855 and DSM 33856 and variants thereof, are preferably able to inhibit the growth of pathogenic *Vibrio parahaemolyticus* strains.

*Escherichia coli* is often the cause of diarrhea in young piglets within a few days of birth until well after weaning. Further, occasional cases of septicemia are also attributable to *E*. *coli.*

*Staphylococcus aureus* subsp. aureus can cause bumblefoot in chickens (McMullin 2004), mastitis in sows (Contreras et al. 2011) and is capable of generating toxins that produce food poisoning in the human body (2016 Centers for Disease Control and Prevention).

Vibrios are known to be associated with disease and high mortality in shrimps, but can also infect finfish. *Vibrio parahaemolyticus* is a marine bacterium that causes seafood borne gastroenteritis and traveler's diarrhea in humans, after consumption of contaminated raw or partially cooked fish or shell fish.

Pathogens can cause further diseases like polyarthritis, fibrinous polyserositis, post-weaning enteric disorders like post-weaning diarrhea and edema disease and swine dysentery.

A further subject of the invention is therefore also a pharmaceutical composition comprising at least one microorganism and/or at least one preparation and/or at least one composition of the invention for treating and/or preventing and/or mitigating the course of a bacterial disease, in particular a bacterial disease as mentioned above.

A very preferred subject in this context is therefore a pharmaceutical composition for treatment and/or prevention and/or mitigating the course of necrotic enteritis, necrohemorrhagic enteritis, necrotic enterocolitis, bacterial enteritis, gangrenous dermatitis, cholangiohepatitis, diarrhea, villous atrophy, foot pad dermatitis, septicemia, meningitis, lameness, arthritis, osteomyelitis and inflammation, in particular, where applicable, also in their sub-clinical forms, in animals, preferably swine or poultry, comprising at least one microorganism and/or at least one preparation and/or at least one composition of the invention.

Another preferred subject in this context is therefore a pharmaceutical composition for treatment and/or prevention and/or mitigating the course of clostridiosis, dyspnea, abdominal distention, scrotal edema, bumblefoot, streptococcal mastitis, polyarthritis, fibrinous polyserositis, post-weaning enteric disorders like post-weaning diarrhea and edema disease, dysentery, inflammation of joints and/or bone tissue and/or of the pericardium, splenomegaly, hepatomegaly, renomegaly, congestion, necrosis, infarction in the liver or spleen, valvular endocarditis, septicemia and/or meningitis, in animals, preferably in swine or poultry, comprising at least one microorganism and/or at least one preparation and/or at least one composition of the invention.

Another preferred subject in this context is also a therapeutic composition for treatment and/or prevention and/or mitigation of the course of a disease of an aquatic animal, in particular of finfish, preferably tilapia, or crustaceans, preferably shrimps, and in particular selected from hemorrhage, comprising at least one microorganism and/or at least one preparation and/or at least one composition of the invention.

A further subject of the invention is therefore likewise the treatment and/or prevention of a disease, in particular of a gut disease, preferably of necrotic enteritis or necrohemorrhagic enteritis, in particular of sub-clinical necrotic enteritis or sub-clinical necrohemorrhagic enteritis, in swine or poultry, wherein at least one microorganism and/or at least one preparation and/or at least one composition of the invention is administered to an animal in need thereof.

A further subject of the invention is therefore likewise the treatment and/or prevention of a disease, preferably a disease of swine or poultry, selected from bacterial enteritis, gangrenous dermatitis, cholangiohepatitis, clostridiosis, diarrhea, dyspnea, abdominal distention, scrotal edema, bumblefoot, foot pad dermatitis, streptococcal mastitis, lameness, arthritis, polyarthritis, fibrinous polyserositis, post-weaning enteric disorders like post-weaning diarrhea and edema disease, dysentery, osteomyelitis, inflammation of joints and/or bone tissue, inflammation of the pericardium, splenomegaly, hepatomegaly, renomegaly, congestion, necrosis, infarction in the liver or spleen, valvular endocarditis, septicemia and/or meningitis, wherein at least one microorganism and/or and least one preparation and/or at least one composition of the invention is administered to an animal in need thereof.

A further preferred subject of the invention is likewise the treatment and/or prevention of a disease of an aquatic animal, in particular of finfish, preferably tilapia, or crustaceans, preferably shrimps, wherein at least one microorganism and/or at least one preparation and/or at least one composition of the invention is administered to an aquatic animal in need thereof.

In a preferred embodiment of the invention, by applying a microorganism and/or a preparation and/or a composition of the invention, in particular a feed composition, the amount of at least one pathogenic bacterium of terrestrial animals, in particular the amount of *Clostridium perfringens, Streptococcus suis* and/or *Enterococcus cecorum,* is reduced at the site of application, in particular in an animal gut, by at least 0.5 log, more preferably by at least 1 log, 2 log, or 3 log.

In a further preferred embodiment of the invention, by applying a microorganism and/or a preparation and/or a composition of the invention, in particular a feed composition, the amount of at least one pathogenic bacterium of aquatic animals, in particular the amount of *Vibrio parahaemolyticus,* is significantly reduced at the site of application, in particular in an animal gut, by at least 0.5 log, more preferably by at least 1 log, 2 log, or 3 log.

As the microorganisms according to the invention are preferably able to bind pathogenic viruses and at the same time inhibit the growth of pathogenic bacteria, thus a preferred embodiment the invention is a therapeutic composition with a broad range of beneficial effects. A further preferred embodiment of the invention is therefore in particular in general a pharmaceutical composition comprising at least one microorganism and/or at least one preparation and/or at least one composition of the invention for treating and/or preventing and/or mitigating the course of a bacterial disease and simultaneously of a viral disease, wherein the bacterial disease and the viral disease are preferably diseases as mentioned before in the description.

The microorganisms of the invention, when administered to animals, preferably enhance the health of such animals and/or improve the general physical condition of such animals and/or improve the feed conversion rate of such animals and/or decrease the mortality rate of such animals and/or increase the survival rate of such animals and/or improve the weight gain of such animals and/or increase the productivity of such animals and/or increase the disease resistance of such animals and/or modulate the immune response of such animals and/or establish or maintain a healthy gut microflora in such animals and/or improve the meat quality of such animals, in particular the meat elasticity and/or the meat hardness, and/or reduce the shedding of bacterial and/or viral pathogens through the feces of such animals. In particular, the microorganisms and compositions of the invention might be used to assist in re-establishing a healthy balance of the gut microflora after administration of antibiotics for therapeutic purposes.

A further subject of the invention is therefore a method of enhancing the health of animals and/or of improving the general physical condition of animals and/or of improving the feed conversion rate of animals and/or of decreasing the mortality rate of animals and/or of increasing the survival rates of animals and/or of improving the weight gain of animals and/or of increasing the productivity of animals and/or of increasing the disease resistance of animals and/or of modulating the immune response of animals and/or of establishing or maintaining a healthy gut microflora in animals and/or of improving the meat quality of animals and/or of reducing the shedding of bacterial and/or viral pathogens through the feces of animals, wherein at least one microorganism and/or at least one preparation and/or at least one composition of the invention is administered to animals.

A further subject of the invention is therefore also a method of enhancing the health of human beings and/or of improving the general physical condition of human beings and/or of increasing the disease resistance of human beings and/or of modulating the immune response of human beings and/or of establishing or maintaining a healthy gut microflora in human beings, wherein at least one microorganism and/or at least one preparation and/or at least one composition according to the invention is administered to human beings.

A further subject of the invention is therefore also the use of at least one microorganism and/or at least one preparation and/or at least one composition of the invention for enhancing the health of animals and/or for improving the general physical condition of animals and/or for improving the feed conversion rate of animals and/or for decreasing the mortality rate of animals and/or for increasing the survival rates of animals and/or for improving the weight gain of animals and/or for increasing the productivity of animals and/or for increasing the disease resistance of animals and/or for modulating the immune response of animals and/or for establishing or maintaining a healthy gut microflora in animals and/or for improving the meat quality of animals and/or for reducing the shedding of bacterial and/or viral pathogens through the feces of animals, wherein the at least one microorganism and/or at least one preparation and/or at least one composition of the invention is administered to animals.

A further subject of the invention is therefore also the use of at least one microorganism and/or at least one preparation and/or at least one compositions of the invention for enhancing the health of human beings and/or for improving the general physical condition of human beings and/or for increasing the disease resistance of human beings and/or for modulating the immune response of human beings and/or for establishing or maintaining a healthy gut microflora in human beings, wherein the at least one microorganism and/or at least one preparation and/or at least one composition of the invention, is administered to human beings.

A further subject of the invention is therefore also at least one microorganism and/or at least one preparation and/or at least one composition of the invention, for enhancing the health of animals and/or human beings and/or for improving the general physical condition of animals and/or human beings and/or for improving the feed conversion rate of animals and/or for decreasing the mortality rate of animals and/or for increasing the survival rate of animals and/or for improving the weight gain of animals and/or for increasing the productivity of animals and/or for increasing the disease resistance of animals and/or human beings and/or for modulating the immune response of animals and/or human beings and/or for establishing or maintaining a healthy gut microflora in animals and/or human beings and/or for improving the meat quality of animals and/or for reducing the shedding of bacterial and/or viral pathogens through the feces of animals.

"Increasing the productivity of animals" refers in particular to any of the following: production of more or higher quality eggs, milk or meat or increased number of offspring or improved survival of offspring.

By using the microorganisms, preparations and compositions of the invention preferably an improvement of at least one of the features as mentioned before is realized, wherein realization of the feature preferably means an improvement of at least 1 %, more preferably of at least 3 or at least 5 %, in comparison to an adequate negative control. As negative control averages known in the animal husbandry field may be used, but preferably as negative control animals which are subjected to the same treatment like the animals tested are used, but without administration of the microorganisms and/or preparations and/or compositions of the invention.

The methods and uses of the microorganisms, preparations and compositions of the invention can be pharmaceutical or non-pharmaceutical, in particular therapeutic or non-therapeutic. In a particularly preferred embodiment of the invention, the methods and uses are non-pharmaceutical, in particular non-therapeutic, preferably feed and food applications.

The microorganisms according to the invention, in particular microorganisms of the genus Bacillus, are preferably further able to increase the growth of at least one beneficial bacterium, in particular in the human or animal gut, wherein the beneficial bacterium is preferably selected from lactic acid bacteria, in particular from lactobacilli and bifidobacteria.

Further the microorganisms according to the invention, i.e. a significant part, in particular at least 80 or 90 %, of a tested sample, preferably survive the high temperatures necessary for pelleting animal feed, in particular they preferably survive a temperature of 80°C, more preferably of 95 or 99°C, for at least 20 minutes.

The microorganisms of the invention may be present, in particular in the compositions of the present invention, as spores (which are dormant), as vegetative cells (which are growing), as transition state cells (which are transitioning from growth phase to sporulation phase) or as a combination of at least two, in particular all of these types of cells. In a preferred embodiment, the compositions of the invention comprise mainly or only spores, wherein preferably at least 60, 70, 80 or 90 % of the microorganisms as contained in the composition are spores.

In addition or as alternative the microorganisms may also be used in non-living, inactivated form, as also the non-living cells are able to bind viral particles. Ways to inactivate the cells are known to those skilled in the art. But as heat-inactivation may lead to a destruction of the glycan structures of the viral decoy receptors, heat-inactivation of the microorganisms is preferably avoided. A big advantage of endospore-forming cells like Bacilli is that the bacterial spores are very heat-resistant, so that the heat-treatment which often occurs in the preparation of feedstuffs, has no big detrimental effect on the Bacilli. Bacilli can then replicate at the site of application, in particular in an animal gut, and produce the viral decoy receptor in situ.

Preferred food compositions according to the invention are fermented foods and/or dairy products, in particular yoghurt, cheese, milk, butter, curd and natto. The microorganisms according to the invention may in particular be used in beverages and in milk replacers, for examples in a baby formula.

The pharmaceutical compositions according to the invention preferably contain at least one pharmaceutically acceptable carrier. They may further contain other ingredients like typical food ingredients as listed further below.

The pharmaceutical composition according to the invention is preferably a liquid, a paste, an aerosol, a suppository or a dried composition, in particular to be administered to the pharyngeal/respiratory tract, to the gastro-intestinal tract, to the urogenital tract or to the skin. Administration might be carried out by using a medical device, for example a spray, a pump or an inhalator.

Thus, a further subject of the present invention is also a method of preparing a pharmaceutical composition, wherein microorganisms according to the invention are mixed with a pharmaceutically acceptable carrier and optionally further compounds and wherein the pharmaceutical composition may be provided in the form of a medical device.

A further subject of the present invention is in particular also a nutritional supplement containing microorganisms according to the invention and at least one pharmaceutically acceptable carrier.

In a preferred embodiment of the invention, the microorganisms of the present invention are administered orally to animals or human beings. The administration is preferably carried out in form of feed and food compositions, but may also be carried out by applying the microorganisms of the invention or preparations thereof to drinking or rearing water. Thus, a further subject of the present invention is water, in particular an aqueous solution or an aqueous suspension, containing at least one microorganism according to the invention or a preparation thereof.

But the microorganisms of the invention can also be used avoid the detrimental effects of viruses outside the animal or human body. Thus, they may be contained and/or applied in sanitizing and/or decontaminant compositions.

Thus, a further subject of the present invention are sanitizing and decontaminant compositions, comprising at least one microorganism according to the invention or a preparation thereof.

Relatedly, the methods of the present invention may be used to decrease the amount of pathogenic bacteria and viruses shed in animal feces.

As the untreated manure of animals due to viral particles, pathogenic bacteria and further ingredients may have a detrimental environmental effect, in particular with respect to the animals themselves and/or with respect to human beings getting in contact with the manure, which can be avoided by either feeding the animals or by treating the manure or the bedding of the animals with the microorganisms of the invention. Therefore a further subject of the invention is a method of controlling and/or avoiding detrimental environmental effects of manure or contaminated liquids, the method comprising the step of applying to manure, contaminated liquids, litter, a pit, or a manure pond at least one microorganism and/or at least one preparation and/or at least one composition according to the invention. Preferably the composition is applied in liquid form, for example by spraying, or as a powder, for example by strewing.

As detrimental viruses and bacteria may have a negative influence on the consistency of litter and in particular may effect a rather fluid litter, which might lead inter alia to foot pad lesions of poultry and which can be avoided by feeding the animals with the microorganisms of the invention, therefore a further subject of the invention is a method of controlling and/or improving the consistency of litter, in particular a method of ensuring a solid consistency of litter and/or a method of avoiding foot pad lesions, the method comprising the step of feeding animals, in particular poultry, with at least one microorganism and/or at least one preparation and/or at least one composition according to the invention.

The microorganisms, preparations and compositions of the invention are very preferably used for improving the quality of water or aqueous solutions. A further subject of the invention is therefore also a method of controlling and/or improving the quality of water or aqueous solutions, in particular of drinking water and/or rearing water and/or effluent water and/or wastewater, comprising the step of applying to water or an aqueous solution at least one microorganism and/or at least one preparation and/or at least one composition of the invention.

As viruses might be distributed in the air and on various surfaces and thus droplet and contact infection might be a special problem in particular for human beings, thus a further subject of the present invention is the use of the microorganisms, preparations and compositions of the invention for treating the air and specific surfaces. Thus, a preferred embodiment of the present invention is also an environmental sanitizer and/or environmental decontaminant and in particular its use for reducing the persistence of viral particles in the environment, preferably to prevent an epidemic spread of the viral particles. Such environments can be in particular surfaces in the hospital environment. The microorganisms may in particular also applied on surfaces of animal facilities or as water quality enhancers in aquaculture either in the open water or in water filters in which the microorganisms are immobilized in biofilms to selectively bind and inactivate virus particles. In another embodiment, the microorganisms can be fed to insects which act as viral vectors to reduce persistence of viral particles in the insect in order to reduce transmission of viral diseases through an insect vector.

Further, the microorganisms, preparations and compositions of the invention can also be used for treating viral infections and/or viral diseases and/or microbial diseases of plants. A further subject of the invention is therefore also a method of treating and/or preventing and/or mitigating the course of diseases of plants, in particular of viral infections and/or viral diseases and/or bacterial diseases of plants, preferably of cultivated plants, comprising the step of applying to the plants at least one microorganism and/or at least one preparation and/or at least one composition of the invention. The application may be carried out in liquid form, such as by spraying, or in solid form, in particular in form of a powder, preferably as a formulated powder.

The compositions of the present invention, in particular the feed, food and pharmaceutical compositions as well as the drinking water, rearing water, effluent water or wastewater, preferably comprise the microorganisms of the invention and are administered to animals at a rate of about 1×10³ to about 2×10¹² CFU/g feed or ml water, in particular in a rate of about 1×10³ or about 1×10⁴ or about 1×10⁵ or about 1×10⁶ or about 1×10⁷ or about 1×10⁸ or about 1×10⁹ or about 1×10¹⁰ or about 1×10¹¹ or about 1×10¹² CFU/g feed or ml water, preferably in an amount of about 1×10⁴ to about 1×10¹⁰ CFU/g feed or ml water, more preferably in an amount of 1×10⁴ to 1×10⁷ CFU/g feed or ml water.

Correspondingly, preferred amounts of the microorganisms and/or preparations of the invention in the feed, food and water compositions of the invention range from 0.01 wt.-% to 10 wt.-%, more preferably from 0.05 wt.-% to 5 wt.-%, in particular from 0.1 wt.-% to 1 wt.-%.

The compositions of the invention may comprise at least one carrier or typical feed ingredients or combinations thereof.

Suitable carriers are inert formulation ingredients added to improve recovery, efficacy, or physical properties and/or to aid in packaging and administration. Such carriers may be added individually or in combination. These carriers may be selected from anti-caking agents, anti-oxidation agents, bulking agents, binders, structurants, coatings and/or protectants. Examples of useful carriers include polysaccharides (in particular starches, maltodextrins, methylcelluloses, gums, chitosan and/or inulins), protein sources (in particular skim-milk powder and/or sweet-whey powder), peptides, sugars (in particular lactose, trehalose, sucrose and/or dextrose), lipids (in particular lecithin, vegetable oils and/or mineral oils), salts (in particular sodium chloride, sodium carbonate, calcium carbonate, chalk, limestone, magnesium carbonate, sodium phosphate, calcium phosphate, magnesium phosphate and/or sodium citrate), and silicates (in particular clays, in particular beolite clay, amorphous silica, fumed/precipitated silicas, zeolites, Fuller's earth, Baylith^{®}, clintpolite, montmorillonite, diatomaceous earth, talc, bentonites, and/or silicate salts like aluminium, magnesium and/or calcium silicate). Suitable carriers for animal feed additives are set forth in the American Feed Control Officials, Inc.' s Official Publication, which publishes annually. See, for example Official Publication of American Feed Control Officials, Sharon Krebs, editor, 2006 edition, ISBN 1-878341-18-9. The carriers can be added after concentrating the fermentation broth and/or during and/or after drying. Preferred carriers according to the invention are selected from calcium carbonate, diatomaceous earth and vegetable oil.

A preferred embodiment of the invention are concentrate compositions, in particular feed additive compositions, i.e. compositions suitable for preparing a feed composition, which comprise at least one microorganism of the invention and at least one carrier as mentioned before, wherein the at least one microorganism is preferably comprised in an amount of 0.1 to 10 wt.-%, more preferably in an amount of 0.2 to 5 wt.-%, in particular in an amount of 0.3 to 3 wt.-%, and the at least one carrier is preferably comprised in an amount of at least 90 wt.-%, preferably in an amount of 90 to 99.9 wt.-%, more preferably in an amount of 95 to 99.8 wt.-%, in particular in an amount of 97 to 99.7 wt.-%, and wherein the carrier consists preferably substantially of limestone, in particular of limestone with smaller parts of diatomaceous earth and/or vegetable oil.

These concentrate compositions can be used for the preparation of feed and pharmaceutical compositions as well as drinking and rearing water which preferably comprise the microorganisms according to the invention in an amount as mentioned in the specification above. In a preferred embodiment 50 to 1000 grams of such a concentrate composition, in particular 50, 100, 250, 500 or 1000 grams of such a concentrate composition, are used per ton of feed, drinking or rearing water to provide compositions which can be used for feeding animals. These concentrate compositions preferably comprise at least one microorganism of the invention in an amount of 1×10⁹ to 2×10¹¹ CFU, in particular 2×10⁹ to 1×10¹¹ CFU, per g of the concentrate composition.

Starting from these concentrate compositions, feed and food compositions can be prepared by mixing the concentrate compositions with typical feed or food ingredients, respectively. The compositions according to the invention, in particular the feed and food composition, preferably contain at least one further feed or food ingredient besides the microorganism according to the invention.

Suitable typical animal feed ingredients which may be contained in the compositions according to the invention and/or used in the preparation of feed compositions starting from concentrate compositions according to the invention include one or more of the following: proteins, carbohydrates, fats, further probiotics, prebiotics, enzymes, vitamins, immune modulators, milk replacers, minerals, amino acids, carriers, coccidiostats, acid-based products and/or medicines, such as antibiotics.

Carbohydrates containing components which may be used according to the invention are for example forage, roughage, wheat meal, corn meal, sunflower meal or soya meal, and mixtures thereof.

Proteins containing components which may be used according to the invention are for example soya protein, pea protein, wheat gluten, corn gluten, rice, canola meal, meal of marine animals, in particular fishmeal, meal of terrestrial animals, and mixtures thereof. "Meal of marine animals" includes meat meal, meat and bone meal, blood meal, liver meal, poultry meal, silkworm meal, silkworm pupae meal and combinations thereof.

Fats are typically provided as oils of marine animals, vegetable oils or oils of microorganisms, in particular oils of microalgae, or combinations thereof. Examples of vegetable oils are soybean oil, rapeseed oil, sunflower seed oil, canola oil, cottonseed oil, flaxseed oil and palm oil. Oils of marine animals include fish oil as well as oil of krill, bivalves, squids or shrimps and further fatty oils from fish of the families Engraulidae, Carangidae, Clupeidae, Osmeridae, Scombridae and/or Ammodytidae. Examples of oils of microalgae are in particular oil of Labyrinthulea, preferably oil of Schizochytria or Thraustochytria. Besides the isolated oils the defatted biomass itself may also be used as fat source, i.e. in particular the meal of a marine animals, preferably fishmeal, or a plant meal, in particular soybean meal, rapeseed meal, sunflower meal, canola meal, cottonseed meal and/or flax seed meal.

Proteins containing components which additionally contain fats which may be used according to the invention are for example fish meal, krill meal, bivalve meal, squid meal or shrimp shells, as well as combinations thereof.

The feedstuff according to the invention has preferably a total protein content of 20 to 50 wt.-% and/or a total fat content of 1 to 15 wt.-% and/or a total carbohydrate content of 20 to 60 wt.-%.

Further probiotics (DFM) and/or microorganisms which may be used according to the invention in combination with the microorganisms and preparations of the invention are preferably bacteria selected from the species *Bacillus subtilis, Bacillus licheniformis, Bacillus paralicheniformis, Bacillus lentus, Bacillus pumilus, Bacillus laterosporus, Bacillus coagulans, Bacillus alevi, Bacillus cereus, Bacillus badius, Bacillus thurigiensis, Bacillus amyloliquefaciens, Bacillus velezensis, Enterococcus faecium,* and *Pediococcus acidilactici.* Preferred examples are *Bacillus pumilus* DSM 32539 and *Bacillus subtilis* DSM 32540 (both deposited with the DSMZ on June 14, 2017 under the provisions of the Budapest Treaty on the International Recognition of the Deposit of Microorganisms for the Purpose of Patent Procedure) and derivatives thereof, *Bacillus licheniformis* DSM 32314 and *Bacillus subtilis* DSM 32315 (both deposited with the DSMZ on May 12, 2016 under the provisions of the Budapest Treaty on the International Recognition of the Deposit of Microorganisms for the Purpose of Patent Procedure) and derivatives thereof, *Bacillus subtilis* PB6 (as described in US Patent No. 7,247,299 and deposited as ATCC Accession No. PTA-6737), which is sold by Kemin under the trademark CLOSTAT^{®}, *Bacillus subtilis* C-3102 (as described in US Patent No. 4,919,936 and deposited as FERM BP- 1096 with the Fermentation Research Institute, Agency of Industrial Science and Technology, in Japan), sold by Calpis as CALSPORIN^{®}, *Bacillus subtilis* DSM 17299, as sold by Chr. Hansen under the trademark GalliPro^{®}, *Bacillus licheniformis* DSM 17236, as sold by Chr. Hansen under the trademark GalliProTect^{®}, a mixture of *Bacillus licheniformis* DSMZ 5749 and *Bacillus subtilis* DSMZ 5750 spores, as sold by Chr. Hansen under the trademark BioPlus^{®}YC, B. *subtilis* DSM 29784, as sold by Adisseo/Novozymes under the trademark Alterion^{®}, *Bacillus subtilis,* as sold by Chr. Hansen under the trademark PORCBOOST^{®}, or *Bacillus coagulans* microorganisms as described in US Patent No. 6,849,256. Other non-Bacillus probiotics, such as *Saccharomyces cerevisiae, Pichia pastoris, Aspergillus niger, Aspergillus oryzae,* or *Hansenula,* may also be used in compositions of the present invention. In particular in food compositions further probiotics which are known to be useful to the human health may be used such as lactic acid producing bacteria, in particular lactobacilli, or Bifidobacteria. If said further probiotics are not formulated as part of the compositions of the present invention, they may be administered together (either at the same time or at different times) with the compositions of the present invention.

Prebiotics which may be used according to the invention are preferably oligosaccharides, in particular selected from galactooligosaccharides, sialyloligosaccharides, lactulose, lactosucrose, fructooligosaccharides, palatinose or isomaltose oligosaccharides, glycosyl sucrose, maltooligosaccharides, isomaltooligosaccharides, cyclodextrins, gentiooligosaccharides, soybean oligosaccharides, xylooligosaccharides, dextrans, pectins, polygalacturonan, rhamnogalacturonan, mannan, hemicellulose, arabinogalactan, arabinan, arabinoxylan, resistant starch, mehbiose, chitosan, agarose, inulin, tagatose, polydextrose, and alginate.

Enzymes which may be used in feed compositions according to the invention and which may aid in the digestion of feed, are preferably selected from phytases (EC 3.1 .3.8 or 3.1.3.26), xylanases (EC 3.2.1.8), galactanases (EC 3.2.1 .89), galactosidases, in particular alpha-galactosidases (EC 3.2.1.22), proteases (EC 3.4), phospholipases, in particular phospholipases A1 (EC 3.1 .1.32), A2 (EC 3.1.1.4), C (EC 3.1.4.3), and D (EC 3.1.4.4), lysophospholipases (EC 3.1 .1.5), amylases, in particular alpha-amylases (EC 3.2.1.1 ); lysozymes (EC 3.2.1 .17), glucanases, in particular beta-glucanases (EC 3.2.1.4 or EC 3.2.1.6), glucoamylases, cellulases, pectinases, or any mixture thereof.

Examples of commercially available phytases include Bio-Feed^{™} Phytase (Novozymes), Ronozyme^{®} P and HiPhos^{™} (DSM Nutritional Products), Natuphos^{™} (BASF), Finase^{®} and Quantum^{®} Blue (AB Enzymes), the Phyzyme^{®} XP (Verenium/DuPont) and Axtra^{®} PHY (DuPont). Other preferred phytases include those described in e.g. WO 98/28408, WO 00/43503, and WO 03/066847.

Examples of commercially available xylanases include Ronozyme^{®} WX and G2 (DSM Nutritional Products), Econase^{®} XT and Barley (AB Vista), Xylathin^{®} (Verenium) and Axtra^{®} XB (Xylanase/beta-glucanase, DuPont). Examples of commercially available proteases include Ronozyme^{®} ProAct (DSM Nutritional Products).

Vitamins which may be used according to the invention are for example vitamin A, vitamin D3, vitamin E, vitamin K, e.g., vitamin K3, vitamin B12, biotin, choline, vitamin B1 , vitamin B2, vitamin B6, niacin, folic acid and pantothenate, e.g. Ca-D-pantothenate, or combinations thereof.

Immmune modulators which may be used are for example antibodies, cytokines, spray-dried plasma, interleukins, or interferons, or combinations thereof.

Minerals which may be used according to the invention are for example boron, cobalt, chloride, chromium, copper, fluoride, iodine, iron, manganese, molybdenum, selenium, zinc, calcium, phosphorous, magnesium, potassium, or sodium, or combinations thereof.

Amino acids which may be used according to the invention are for example lysine, alanine, threonine, methionine or tryptophan, or combinations thereof.

The feedstuffs of the invention may further comprise betaine and/or choline and/or other physiologically effective methyl group donors. The feedstuffs may further comprise polyunsaturated fatty acids, in particular DHA and/or EPA.

Thus, a further embodiment of the invention is also a method of preparing an animal feed composition comprising mixing at least one microorganism and/or at least one preparation and/or at least one concentrate composition of the invention, in particular in an amount effective to enhance animal health, in particular gut health, with feed ingredients, such as proteins, lipids and/or carbohydrates, and optionally further beneficial substances, preferably as mentioned before, to provide a feeding product. This method may comprise for example also a pelleting step.

Standard pelleting processes known to those of skill in the art may be used, including extrusion processing of dry or semi-moist feeds. Preferred pelleting temperatures are between about 65° C and about 120° C.

The microorganisms and/or preparations and/or compositions of the invention can be administered to animals in feed and/or drinking water and/or rearing water over multiple days throughout the animal's life or during particular stages or portions of the animal's life. For example, the microorganisms and/or preparations and/or compositions can be administered only in a starter diet or only in a finisher diet of farm animals.

The microorganisms, preparations and compositions of the invention can further be employed in a wide dosage range. Daily doses are, for example, in the range of between approximately 1 mg and approximately 500 mg, in particular in the range of approximately 5 mg and approximately 200 mg, in the range of from approximately 10 mg to approximately 100 mg or in the range of from approximately 20 to approximately 60 mg per kilogram live weight.

Preferably according to the invention always an effective amount of the microorganisms and/or preparations and/or compositions of the invention is used in the embodiments of the invention. The term "effective amount" refers to an amount which effects at least one beneficial effect to an animal and/or to the environment, in particular with respect to the features as already mentioned before, in comparison to an animal or environment that has not been administered the microorganisms and/or preparations and/or compositions of the invention, but besides that has been administered the same diet (including feed and other compounds) or the same composition, respectively.

In case of therapeutic applications preferably a therapeutic amount of the microorganisms and/or preparations and/or compositions of the invention is used. The term "therapeutic amount" refers to an amount sufficient to ameliorate, reverse or prevent a disease state in an animal. Optimal dosage levels for various animals can easily be determined by those skilled in the art, by evaluating, among other things, the composition's ability to (i) inhibit or reduce pathogenic viruses and/or bacteria in the gastrointestinal tract, in particular in the gut, or in the oral or nasopharyngeal cavity or on the skin or in the gills at various doses, (ii) increase or maintain levels of beneficial bacteria and /or (iii) enhance animal health, in particular gut health, at various doses.

The methods of the present invention may be used for all kind of animals, in particular all kind of vertebrates such as mammals, aquatic animals and birds.

Animals that may benefit from the invention include but are not limited to farm animals, pets, exotic animals, zoo animals, animals used for sports, recreation or work.

In a very preferred embodiment of the invention, the animals are farm animals, which are raised for consumption or as food-producers, such as poultry, swine and ruminants.

The poultry may be selected from productive or domestic poultry, but also from fancy poultry or wild fowl. Preferred productive poultry in this context are chickens, turkeys, ducks and geese. The productive livestock in this context is preferably poultry optimized for producing young stock or poultry optimized for bearing meat. Preferred fancy poultry or wild fowl are peacocks, pheasants, partridges, chukkars, guinea fowl, quails, capercaillies, grouse, pigeons and swans, with quails being especially preferred. Further preferred poultry are ratites, in particular ostriches and emus, as well as parrots.

Ruminants according to the invention are preferably selected from cattle, goat and sheep. In one embodiment, the compositions of this invention may be fed to preruminants to enhance their health and, in particular, to decrease the incidence of diarrhea in these animals. Preruminants are ruminants, including calves, ranging in age from birth to about twelve weeks.

Pets are preferably selected from dogs, cats, domestic birds and domestic exotic animals. Animals used for sports may in particular be horses.

The aquatic animals according to the invention are preferably finfish, in particular of the class Actinopterygii, or crustaceans. Actinopterygii include, in particular, tilapia and other cichlids, carps and other cyprinids, salmons and other salmonids, catfish, in particular African catfish and pangasius, tuna, perch, cod, smelt, milkfish, gourami, seabass, in particular barramundi, seabream, grouper and snakehead fish.

Preferred types of salmon and salmonids in this context are the Atlantic salmon, red salmon (sockeye salmon), masu salmon (cherry salmon), king salmon (Chinook salmon), keta salmon (chum salmon), coho salmon, Danube salmon, Pacific salmon, pink salmon and trout. The aquatic animals according to the invention are very preferred tilapia.

Crustaceans include in particular shrimps, lobster, crabs, prawns and crayfish.

Preferred types of shrimps in this context are *Litopenaeus, Farfantepenaeus* and *Penaeus,* in particular *Penaeus stylirostris*, *Penaeus vannamei*, *Penaeus monodon*, *Penaeus chinensis*, *Penaeus occidentalis, Penaeus calif orniensis*, *Penaeus semisulcatus, Penaeus esculentu, Penaeus setiferus*, *Penaeus japonicus*, *Penaeus aztecus*, *Penaeus duorarum*, *Penaeus indicus*, and *Penaeus merguiensis.* Very preferred according to the invention is the shrimp *Penaeus vannamei,* also called whiteleg shrimp.

The aquatic animals, and in particular the shrimp, which are treated or fed with the microorganisms and/or preparations and/or compositions according to the invention can in particular be larvae, post-larvae or juvenile shrimp.

The aquatic animals may in particular also be fish which is subsequently processed into fish meal or fish oil. In this connection, the fish are preferably herring, pollack, cod or small pelagic fish like anchovy, blue whiting, capelin, driftfish, jack, mackerel, menhaden, sardine or scad fish. The fish meal or fish oil thus obtained, in turn, can be used in aquaculture for farming edible fish or crustaceans.

The aquatic animals may further be oysters, clams, cockles, arkshells, bivalves, mussels or scallops.

However, the aquatic animals may also be small organisms which are used as feedstuff in aquaculture. These small organisms may take the form of, for example, nematodes, small crustaceans or rotifers.

The farming of aquatic animals may take place in ponds, tanks, basins or else in segregated areas in the sea or in lakes or in rivers, in particular in this case in cages or net pens. Farming may be used for farming the finished edible fish, but also may be used for farming fry which are subsequently released so as to restock the wild fish stocks.

In salmon farming, the fish are preferably first grown into smolts in freshwater tanks or artificial watercourses and then grown on in cages or net pens which float in the sea, ponds or rivers and which are preferably anchored in bays or fjords.

Accordingly, the feedstuff according to the invention is preferably a feedstuff for use in the farming of the above-mentioned animals. Preferred applications for animal feed according to the invention is in the feed of cattle, swine, poultry, fish, crustacean or companion animals, with swine and poultry being particularly preferred. Spore forming microorganisms, in particular of the genus *Bacillus,* are particularly suited for this application as they produce heat resistant spores which can be mixed into feed prior to the pelleting process of animal feed. Microorganisms according to the invention are very preferably used in milk replacers for cattle, goats, sheep and swine or as post-pelleting spray-on coating for pelleted feed material.

A further subject of the present invention is a method of identifying microorganisms according to the invention.

The binding of viral particles to the microorganisms according to the invention can only be assessed directly for viruses which can be produced in sufficient amount in cell culture, which is challenging for most virus strains. Furthermore, the production of infectious viral particles and their use in binding assays poses biological risks. Thus, an alternative method for identifying microorganisms according to the invention had to be applied, which is a further subject of the present invention.

Thus, a further subject of the invention is a method of identifying microorganism which display viral decoy receptors on the surface, comprising the following steps:
a) Heterologous expression and purification of viral receptor binding proteins, in particular virolectins, and their assembly on a carrier;
b) Exposing the carriers carrying the viral receptor binding proteins to a collection of microorganisms;
c) Examining whether microorganisms are bound to the viral particles on the carrier by centrifugation of the microorganisms and examination of the residual, unbound, nanoparticles in the supernatant.

The carrier is preferably a particle, in particular a nanoparticle. The particle or nanoparticle is preferably a protein.

The viral particles or parts thereof, in particular viral proteins or virolectins, can be immobilized on the carrier in different ways. In particular the viral proteins or virolectins might be immobilized on the carrier by binding them to protein particles by covalent bonding or non-covalent bonding. Covalent bonding can be realized for example by heterologously expressing fusion proteins of viral proteins, in particular virolectins, with other proteins which form particles.

In a preferred embodiment of the invention viral proteins are produced as fusion proteins with larger proteins. Very preferably they are produced as fusion proteins with lumazine synthase. In a very particularly preferred embodiment of the invention the lectin VP8* or parts thereof are produced as fusion protein, preferably with lumazine synthase, and thus form particles which display VP8* or parts thereof.

In a further preferred embodiment of the invention the viral proteins are bound by van der Waals bonding to fusion proteins which have been produced before. Very preferably the fusion protein, to which the viral proteins, in particular virolectins, are bound are fusion proteins of protein A and lumazine synthase.

Using viral receptor-binding proteins, microorganisms according to the invention can be identified in a pure culture. Hereby, the use of viral receptor-binding proteins allows to probe for the presence of viral-decoy receptors and simultaneous verification of virus-binding functionality.

The microorganisms, compositions and preparations of the present invention can be obtained by culturing the microorganisms of the invention according to methods well known in the art, including by using the media and other methods as described for example in US 6,060,051, EP0287699 or US2014/0010792. Conventional large-scale microbial culture processes include submerged fermentation, solid state fermentation, or liquid surface culture. Towards the end of fermentation, as nutrients are depleted, the cells of the strains preferably begin the transition from growth phase to sporulation phase, such that the final product of fermentation is largely spores, metabolites and residual fermentation medium. Sporulation is part of the natural life cycle of *Bacillus* cells and is generally initiated by the cell in response to nutrient limitation. Fermentation is configured to obtain high levels of colony forming units of the microbial cells, in particular *Bacillus* cells, and preferably to promote sporulation. The bacterial cells, spores and metabolites in culture media resulting from fermentation may be used directly or concentrated by conventional industrial methods, such as centrifugation, tangential-flow filtration, depth filtration, and evaporation. The concentrated fermentation broth may be washed, for example via a diafiltration process, to remove residual fermentation broth and metabolites.

According to the invention, the term "preparation(s)" refers with respect to the microorganisms of the invention to any kind of preparation, which contains the microorganisms of the invention or debris thereof or viral decoy receptors thereof or any mixtures thereof; but besides that the term "preparation(s)" refers also to cell-free preparations and in particular to preparations which are even free of cell debris, but which contain substances which were produced by the microorganisms of the invention, wherein such preparations can be obtained for example either starting from a dried or suspended biomass containing the microorganisms or from a fermentation broth, which is obtained by cultivating the microorganisms in a fermentation medium.

The fermentation broth or broth concentrate, containing the microorganisms of the invention, can be dried with or without the addition of carriers using conventional drying processes or methods such as spray drying, freeze drying, tray drying, fluidized-bed drying, drum drying, or evaporation. The resulting dry products may be further processed, such as by milling or granulation, to achieve a specific particle size or physical format. Carriers, as described above, may also be added post-drying.

As the viral decoy receptors are located on the cell surface of the microorganisms according to the invention, thus cell-containing preparations of the microorganisms of the invention are more preferred. But as the microorganisms of the invention produce and secret further beneficial compounds like anti-pathogenic metabolites and enzymes, which are still active also in the absence of the cells, the cell-free preparations are a further embodiment of the present invention.

Cell debris of the microorganisms of the invention are principally still able to bind viruses, as breaking the cells has not necessarily an impact on the viral decoy receptors which are localized on the cell surface. Further, the viral decoy receptors can be removed from the cell surface of the microorganisms enzymatically, in particular by applying a neuraminidase, so that cell-free preparations which are also free of cell debris can be prepared which still contain active viral decoy receptors, so that also cell-free preparations of the microorganisms may still be used to inhibit viruses.

Cell-free preparations of the microorganisms of the invention can be obtained for example by centrifugation and/or filtration of a fermentation broth, optionally after applying a neuraminidase. Depending on the technique used, preparations which are obtained this way may not be completely devoid of cells, but may still comprise a smaller amount of cells. As the cells secret compounds like metabolites, enzymes and/or peptides into the surrounding medium, the supernatant of the cells comprises a mixture of such compounds, in particular metabolites, enzymes and/or peptides, as secreted by the cells, and optionally viral decoy receptors as obtained by applying a neuraminidase, before removing the cells and cell debris. Thus, in a preferred embodiment of the invention, the preparation of the microorganisms of the invention is a supernatant of the fermentation broth. The preparation of the microorganisms, in particular the supernatant, can also be used in dried form, wherein drying can be carried out for example by spray-drying or freeze-drying.

Preparations comprising cell debris of the microorganisms may be obtained by rupturing the cells applying techniques as known to those of skill in the art, for example by mechanical means or by applying high pressure. Depending on the degree of force applied, a preparation comprising only ruptured cells or a composition comprising a mixture of cell debris and intact cells is obtained. Homogenization of the cells may be realized for example by utilizing a French cell press, sonicator, homogenizer, microfluidizer, ball mill, rod mill, pebble mill, bead mill, high pressure grinding roll, vertical shaft impactor, industrial blender, high shear mixer, paddle mixer, and/or polytron homogenizer. Suitable alternatives are enzymatic and/or chemical treatment of the cells.

Cell-free preparations of the invention comprise also preparations which are obtained by first rupturing the cells by applying techniques as mentioned before and subsequently removing the cell debris and the remaining intact cells. Removing of the cell debris and remaining intact cells can be carried out in particular by centrifugation and/or filtration.

The preparations of the microorganisms of the invention may comprise as active compounds at least one metabolite, preferably a mixture of metabolites, as further described below, and/or at least one enzyme selected from proteases, xylanases and/or cellulases, and/or at least one peptide, and/or viral decoy receptors, in particular Sia-containing viral decoy receptors and/or combinations thereof.

The preparations according to the invention preferably possess the same characteristics like the microorganisms according to the invention, in particular like the strains DSM 33585, DSM 33586, DSM 33587 or DSM 33588, with exception of the characteristics which are intrinsic to intact cells like the ability to grow, in particular under challenging conditions. In particular, the preparations of the invention are preferably able to inhibit the growth of at least one pathogenic bacterium, preferably the growth of at least one, in particular at least two, more preferably of all, of the pathogenic bacteria *Clostridium perfringens*, *Streptococcus suis* and *Enterococcus cecorum.*

A preparation containing an effective mixture of metabolites as contained in the microorganisms of the invention and/or as contained in the cell preparations as mentioned before, can be obtained for example according to the methods set forth in US Patent No. 6,060,051. In particular the preparation can be obtained by precipitating the metabolites as contained in the preparations mentioned before by using organic solvents like ethyl acetate and subsequent redissolving of the precipitated metabolites in an appropriate solvent. The metabolites may subsequently be purified by size exclusion filtration that groups metabolites into different fractions based on molecular weight cutoff.

The preparation, in particular cell-free preparation, containing an effective mixture of metabolites of the invention preferably comprises at least five, more preferably at least 6, 7, 8, 9 or 10, in particular all metabolites of the respective microorganisms of the invention. The metabolites possess preferably a molecular weight of between 400 and 5000 Dalton, more preferably of between 800 and 4000 Dalton. The preparation preferably further comprises viral decoy receptors, in particular Sia-containing viral decoy receptors.

### Working examples

### Example 1: Virus strain selection

For PEDV, the Sia-binding strain GDU was used (Li et al. (2016): Virus Res. 226, 117-127). In the case of rotavirus group A viruses (RAV), representative variants of three porcine P-genotypes, P[7] strain OSU (ATCC VR-892), p[6] strain Z84, and P[19] strain Mm7, were selected based on i. published evidence of the bacterially expressed VP8*-core binding to glycans, ii. availability of VP8*-glycan holostructures, and iii. geno- and phenotypic differences to account for RAV diversity and potentially correlating with differences in glycan receptor usage (Liu et al. (2017): PLoS Pathog. 13; Sun et al. (2018). J. Virol. 92). The P[6] and P[19] strains were previously identified as non-Sia binders. The P[7] Osu was selected for its well described binding to sialosides but also because this strain is available at the American Tissue Culture Collection and can be propagated in cultured cells.

### Example 2: Propagation of cells and viruses

African green monkey kidney cells (Vero-CCL81), pig kidney epithelial cells (LLCPK1), human embryonic kidney 293 cells stably expressing the SV40 large T antigen (HEK-293T) were maintained in Dulbecco modified Eagle medium (DMEM, Lonza) supplemented with 10% fetal bovine serum (FBS) and 1% penicillin/streptomycin (pen/strep; HyClone). Epithelial Monkey Kidney Cells (MA104) were maintained in Eagle's minimal essential medium (EMEM, Lonza) supplemented with 10% FBS.

RAV strain OSU (obtained from ATCC) was propagated in MA104 cells in EMEM, supplemented with 0.5 µg/mL TPCK-treated trypsin Gibco trypsin (Invitrogen). PEDV-GDU was propagated and titrated in Vero-CCL81 cells in DMEM supplemented with 1µg/mL TPCK-treated trypsin (Sigma-Aldrich).

### Example 3: Production of PEDV-GDU S1-Fc and multivalent presentation on protein A-tagged lumazine synthase nanoparticles

PEDV-GDU S1-Fc and protein A-tagged lumazine synthase (pA-LS) nanoparticles were produced separately by transient expression in HEK293T cells as described (Li et al. (2016) Virus Res. 226, 117-127; Li et al. (2017) Proc. Natl. Acad. Sci. 114, E8508-E8517). For PEDV-GDU S1-Fc, a pCAGGS-based expression vector was used, encoding a chimeric product comprised of residues 1-729 of the S protein and the Fc domain of human IgG1, separated by a thrombin cleavage site.

Lumazine synthase (LS) nanoparticles for multivalent presentation of PEDV-GDU S1-Fc were expressed from a pCAGGS-based plasmid encoding the 154-residue-long lumazine synthase protein of the hyperthermophile bacterium *Aquifex aeolicus*, N-terminally extended with a CD5 signal peptide (to ensure secretion into the tissue culture supernatant), a strep tag (to facilitate protein purification and allow detection in ELISA), and the 59residue-long domain B of protein A (pA) of *Staphylococcus aureus* (Li et al. (2016): Virus Res. 226, 117-127; Li et al. (2017): Proc. Natl. Acad. Sci. 114, E8508-E8517).

For protein production, HEK293T cells (~2*10⁷) were transfected with a mixture of 20µg plasmid DNA and 2 µg polyethyleneimine (Polysciences) in 800 µl Opti-MEM^{™} (Thermo Scientific). At 16 h post transfection, the transfection mixture was replaced by 293 SFM II expression medium (Invitrogen), supplemented with 44.1 mM sodium bicarbonate, 11.1 mM glucose, Primatone RL-UF (3.0 g/l), penicillin (100 IU/ml), streptomycin (100 µg/ml), 1% glutaMAX (Gibco), and 1.5% (v/v) DMSO. Cell supernatants were harvested 6 to 7 days after transfection and clarified by consecutive centrifugation at 1200rpm, 4°C for 5 min and 4000 rpm, 4°C for 10 min. For PEDV S1-Fc purification, 250 µl 50% Protein A-Sepharose in PBS (v/v) was added per 50 ml supernatant, followed by overnight incubation at 4°C and collection through a Poly-Prep chromatography column (Bio-rad). PEDV S1-Fc was eluted with 0.1M citric acid (with a volume equivalent to that of the Protein A-Sepharose) and neutralized immediately with 1/3 volume of 1M Tris-HCI, pH8.8.

For pA-LS purification, 200 µl StrepTactin Sepharose beads (IBA) were added per 50 mL supernatant, followed by overnight incubation at 4°C and collection through a Poly-Prep chromatography column (Bio-rad). Elution was with strep elution buffer according to the manufacturers' instruction (IBA).

The PEDV S1-Fc and pA-LS preparations were dialyzed against PBS for 16 hr, protein concentrations were determined by Nanodrop^{™} 1000 (Thermo Scientific) spectrophotometry, and samples of the purified proteins were routinely analyzed by SDS-PAGE, HAA and/or sp-LBA for quality control and to standardize adsorption assays. The materials were aliquoted and stored at - 80°C until use.

Nanoparticles multivalently presenting the PEDV S1 virolectin were freshly produced, immediately before use, by mixing PEDV S1-Fc and pA-LS at a 0.6:1 molar ratio for 30 min at room temperature in PBS.

### Example 4: Production of RAV VP8*-core-LS fusion proteins.

Porcine RAV VP8*-based virolectins were produced by bacterial expression as described (Sun et al. (2018): J. Virol. 92). To this end, the synthetic E. coli-optimized coding sequences for the VP8* core domain (VP4 residues 64-223) of P[7] strain OSU, p[6] strain Z84, and P[19] strain Mm7, genetically fused to coding sequences for lumazine synthase and a strep tag, were cloned in expression vector pGEX2T (Appendix, sFig. 3-5). The p[6] FYNS mutant (HGGR¹⁶⁹⁻¹⁷²FYNS, Sun et al. (2018): J. Virol. 92) and p[19] Arg²⁰⁹Ala mutants were generated by site-directed mutagenesis of the p[6] or p[19] expression vector using the Q5 kit (New England Biolabs). For protein production, competent E. coli BL21 cells (~10⁶) were subjected to transformation with 10 ng plasmid DNA and grown for 16 hr at 37°C in 5 ml LB broth containing 100µg/ml ampicillin (LB-Amp). Then, 400 ml LB-amp was added and incubation at 37°C continued until bacterial cultures reached an optical density of 0.5 measured at 600 nm upon which isopropyl β- d-1-thiogalactopyranoside (IPTG, Sigma-Aldrich) was added to a final concentration of 500 µM. After an additional 3 to 4 hr incubation at 37°C, bacteria were pelleted by centrifugation (3000 X g, 15 min, 4°C) and lysed by 4 consecutive on-off sonication cycles (Branson 450 Digital Sonifier, Marshall Scientific). The lysates were centrifuged for 3000 X g, 30 min, 4°C to remove insoluble debris. The supernatants were collected and incubated overnight at 4°C with StrepTactin Sepharose beads (IBA GmbH; 200 µl per 10ml lysate). Beads were collected with a Poly-Prep chromatography column (Bio-rad), and VP8*-LS proteins were eluted with strep elution buffer as per the manufacturers' instruction and dialyzed against PBS, analyzed, aliquoted and stored at -80°C.

### Example 5: Hemagglutination assay (HAA)

HAA was performed according to standard procedures to detect and quantitatively assess virus or virolectin binding to sialosides. Human, porcine or chicken EDTA blood samples were centrifuged (300 X g, 10 min, 4 °C) to collect the erythrocytes. The cells were then washed five times with PBS and finally suspended in PBS to final concentrations (v/v) of 0.5%. Of these erythrocyte suspensions, 50µl was added to 50µl of two-fold serial dilutions in PBS of virus preparations (PEDV, RAV-OSU) or virolectins (PEDV-GDU S1-Fc-loaded pA-LS nanoparticles or purified RAV VP8*-LS) in 96-well, V-bottom plates (Greiner Bio-One). Incubation was on ice for at least 2 hr. HAA titers were scored once non-agglutinated erythrocytes were completely settled on the bottom of the wells.

To confirm virus or virolectin binding to cell surface Sia, erythrocytes suspended in PBS were desialylated with 20 mU/ml neuraminidase (*A*. *ureafaciens*; Roche) for 3 hours at 37 °C and then washed three times, resuspended to 0.5% in PBS and used for HAA, in parallel with non-treated erythrocytes, as described above.

### Example 6: Solid-Phase Lectin-Binding Assay (sp-LBA)

To detect and quantitatively assess binding of VP8*-LS virolectins of non-Sia-dependent RAVs p[6] Z84 and p[19] Mm7, a modified ELISA assay, sp-LBA was developed, with porcine gastric mucin (PGM; Sigma-Aldrich) as glycoconjugate. PGM mostly contains core 1 and core 2 glycans, mainly terminated by galactose and with low levels of sialylation. Nunc Maxisorp^{™} 96-well, flat-bottom plates (Thermo Scientific) were coated with PGM (0.1 mg/ml porcine gut mucin (Sigma-Aldrich) in PBS; 100 µl/well) for 16 hours at 4 °C. Plates were washed three times with washing buffer (PBS + 0.05% Tween-20) and subsequently blocked for 2 h at 37 °C with 200 µl of blocking buffer (PBS + 0.05% Tween-20, containing 2% Bovine Serum Albumin). Plates were stored at -20°C and washed three times with washing buffer before use.

Binding assays were performed with 100 µl of twofold serial dilutions of VP8*-LS in blocking buffer starting at 2.5 µg/ml. Incubation was for one hour at 37 °C, after which plates were washed three times with washing buffer, and bound VP8*-LS proteins were detected using the HRP-conjugated anti-StrepMAB antibody (IBA GmbH; 1:2000 diluted in blocking buffer; 100 µl/well; 30 min incubation) that recognizes their N-terminal streptomycin tag. Lastly, to measure bound HRP activity, 100µl TMB super slow (Sigma-Aldrich) was added per well and after 10 incubation, the reaction was quenched with 12.5% sulfuric acid and optical density (OD) was measured at 450 nm with an ELx808 ELISA microplate reader (BioTek).

### Example 7: Screening of bacterial strain by virolectin/virus adsorption assays

A library of about 200 Bacillus strains was screened batchwise for adsorption of virolectins (PEDV-GDU S1-Fc-loaded pA-LS nanoparticles or purified RAV VP8*-LS). In the case of PEDV, the observations were corroborated by adsorption assays with virus particles. Bacteria were grown in LB medium for 16 hr at 37°C. Of these cultures, bacteria from 0.5 ml samples (an estimated 10⁷ cells; assuming that an OD600 of 0.65 corresponds with 2×10⁷ cfu/ml) were pelleted at 5000 × g for 1 min at room temperature, washed once with 1 ml ice-cold PBS, pelleted again, and resuspended in 200 ul PBS, containing fixed amounts of virolectin. The non-Sia-binding RAV P[6] and P[19] virolectin, with spLBA as read-out, were diluted to 2.5 ng/ul (in total 250 ng of each virolectin preparation/reaction). For Sia-binding virolectins (PEDV-GDU S1-Fc-loaded pA-LS nanoparticles or purified RAV P[7] VP8*-LS), the equivalent of 200 hemagglutinating units (HAUs; as determined by standard HAA) was added. For adsorption assays with PEDV virus particles, the experiment was performed as described except that for each reaction the equivalent of 128 HAUs was used (corresponding to 2.10⁶ plaque-forming units (PFU) and at particle-PFU ratios of up to 1:1000 as observed for other coronaviruses, up to 2.10⁹ physical particles). Incubation of bacterial cells and virolectins or PEDV was for 30 min on ice. The bacteria were then pelleted and adsorption of virolectin was detected by measuring the lectin activity remaining in the supernatants, by sp-LBA or standard HAA with human erythrocytes as described above. The results were presented in percentages calculated from the input amount of virolectin and the percentual amount of virolectin remaining in the supernatant after adsorption.

Adsorption assays were performed with desialylated or with killed, formaldehyde-fixed bacteria. To deplete cell surface Sias, live bacteria were treated with 20 mU/mL *Arthrobacter ureafaciens* (Roche) or *Vibrio cholerae* neuraminidase NA (Roche) in PBS for 3 hours at 37 °C and washed 3 times with ice-cold PBS, resuspended to 50% and kept on ice. Formaldehyde fixation was performed by treating live bacteria with 3.7% paraformaldehyde (Merck KGaA) in PBS for 30 min at room temperature. After fixation, bacteria were washed 3 times with ice-cold PBS, resuspended to 50% and kept on ice.

To measure relative binding capacity, bacterial cultures were diluted to an OD600 of 0.5. Of these calibrated samples, 2-fold dilution serial dilutions were tested by virolectin adsorption assay as above.

Thus, by screening about 200 naturally occurring Bacillus strains, 27 were identified to be able to bind to viral PEDV particles. It could further be shown that binding is not affected by temperature and pH, as binding took place also at a temperature of 37°C and at a pH range from 6.0 to 9.0. By showing that PEDV particles were not able to bind to the desialylated neuraminidase-treated Bacillus strains, it could be shown that binding takes place to sialic acid or to sialic acid containing binding sites on the Bacillus strains. The identified binding strains were of the species *Bacillus subtilis*, *Bacillus velezensis, Bacillus pumilus* and *Bacillus megaterium*. 16 of those 27 strains were able to bind in addition to viral VP8* particles. Of the identified strains, four were selected, because they exhibited some further positive characteristics which identified them as particularly suitable for feed applications. The selected strains were deposited at the DSMZ (Deutsche Sammlung für Mikroorgansimen und Zellkulturen, Inhoffenstraße 7B, 38124 Braunschweig, Germany) and obtained the following deposit numbers: DSM 33855, DSM 33856, DSM 33857 and DSM 33858. Of the four deposited strains, three are of the species *B*. *subtilis* (DSM 33855, DSM 33856 and DSM 33858), while one belongs to the species *B*. *velezensis* (DSM 33857).

### Example 8: Determination of antimicrobial susceptibility of the identified strains

After elimination of pathogens from the collection of isolates, all isolates with potential application as probiotics/DFM were screened for the presence of antibiotic resistances. In the following, the determination of the minimal inhibitory concentration (MIC) for the Bacillus strains DSM 33855, DSM 33856, DSM 33857 and DSM 33858 with respect to a selected group of 12 antimicrobials according to the "Guidance on the characterization of microorganisms used as feed additives or as production organisms" of the European Food Safety Authority (EFSA, 2018) is shown. Based on the EFSA Guidance, the risk of adding new antimicrobial resistances to the pool already present should be minimized by only applying microorganisms not exhibiting other than intrinsic resistances to antimicrobials critically or highly important for human and animal treatment.

The MIC tests were carried out by applying the broth microdilution method with a panel of 12 selected antimicrobial compounds according to Clinical and Laboratory Standards Institute M07-A9 (2012) and ISO 20776-1 (2018) with *Staphylococcus aureus* ATCC 29213 as CLSI quality control (QC) strain.

**Table 1: Overview on MIC values for Bacillus strains DSM 33855, DSM 33856, DSM 33857, DSM 33858 for 6 antibiotics with categorization in sensitive (S) or resistant (R) according to EFSA cutoff**

| Strain | Chloramphenicol (µg/ml) | Vancomycin (µg/ml) | Erythromycin (µg/ml) | Streptomycin (µg/ml) | Clindamycin (µg/ml) | Tetracycline (µg/ml) |
|---|---|---|---|---|---|---|
| EFSA Cutoff Bacillus | 8 | 4 | 4 | 8 | 4 | 8 |
| DSM 33855 | 4 (S) | 0.5 (S) | 0.25 (S) | 16 (S) | 0.25 (S) | 4 (S) |
| DSM 33858 | 4 (S) | 0.5 (S) | 0.25 (S) | 16 (S) | 0.25 (S) | 8 (S) |
| DSM 33856 | 4 (S) | 0.25 (S) | >0.125 (S) | 4 (S) | 0.5 (S) | >0.25 (S) |
| DSM 33857 | 4 (S) | 0.5 (S) | >0.125 (S) | 2 (S) | 1 (S) | 8 (S) |

**Table 2: Overview on MIC values for Bacillus strains DSM 33855, DSM 33856, DSM 33857, DSM 33858 for 6 further antibiotics with categorization in sensitive (S) or resistant (R) according to EFSA cutoff**

| Stamm | Gentamycin (µg/ml) | Ampicillin (µg/ml) | Nourseothricin (µg/ml) | Daptomycin (µg/ml) | VirginiamycinM1 (µg/ml) | Kanamycin (µg/ml) |
|---|---|---|---|---|---|---|
| EFSA Cutoff Bacillus | 4 | NA | NA | NA | NA | 8 |
| DSM 33855 | >0.076 (S) | >0.125 (S) | <0.25 (S) | 2 (S) | 4 (S) | 1 (S) |
| DSM 33858 | 0.152 (S) | >0.125 (S) | <0.25 (S) | 2 (S) | 2 (S) | 1 (S) |
| DSM 33856 | >0.076 (S) | >0.125 (S) | <0.25 (S) | 2 (S) | 2 (S) | 0.5 (S) |
| DSM 33857 | >0.076 (S) | 0.5 (S) | <0.25 (S) | 2 (S) | 2 (S) | 0.5 (S) |

| | | | | | | |
|---|---|---|---|---|---|---|
| NA: EFSA cutoff not provided for Bacillus | | | | | | |

The Bacillus strains DSM 33856 and DSM 33857 are sensitive to all 12 tested antibiotics, i.e. they are sensitive to Chloramphenicol, Vancomycin, Erythromycin, Streptomycin, Clindamycin, Tetracycline, Gentamycin, Ampicillin, Kanamycin, Nourseothricin, Daptomycin and Virginiamycin M1.

The strains DSM 33855 and DSM 33858 are sensitive to all tested antibiotics with exception of streptomycin, as they have a MIC value of 16 with respect to streptomycin, which is double the given EFSA MIC of 8. But this method is considered to be unprecise by up to plus or minus one two-fold. Thus, this MIC value regarding streptomycin should rather also be considered as still tolerable.

### Example 9: Pathogen Inhibition of identified strains

An initial screening showed that the group of 27 identified *B*. *subtilis, B. megaterium, B. velezensis* and *B. pumilus* strains which displayed viral decoy receptors comprised microorganisms which were able to inhibit the growth of pathogenic bacteria which are relevant for livestock farming. The pathogen inhibition results of the preferred strains DSM 33855, DSM 33856, DSM 33857 and DSM 33858 are disclosed in the following.

A well diffusion antagonisms test was performed with different pathogenic strains of the species *Clostridium perfringens*, *Salmonella enterica* subsp. enterica, *Streptococcus suis*, *Escherichia coli*, *Enterococcus cecorum* and *Vibrio parahamolyticus.*

Strain pathogenic C. perfringens ATCC 13124 is known to be alpha-toxigenic Type A strain serving as a type strain for Clostridia and is also used by Teo and Tan (2005).

S. *suis* is an important pathogen in pigs and one of the most important causes of bacterial mortality in piglets after weaning causing septicemia, meningitis and many other infections [Goyette-Desjardins et al. 2014]. DSM9628 belongs to Serotype 3 and was isolated from Pleural fluid.

*E. cecorum* is known to cause lameness, arthritis and osteomyelitis in broilers usually caused by an inflammation of a joint and/or bone tissue. Further *E*. *cecorum* can cause an inflammation of the pericardium.

Vibrios are known to be associated with disease and high mortality in shrimps, but can also infect finfish. *Vibrio parahaemolyticus* is a marine bacterium that causes seafood borne gastroenteritis and traveler's diarrhea in humans, after consumption of contaminated raw or partially cooked fish or shell fish.

Diarrhea associated with *Escherichia coli* can occur in young piglets within a few days of birth until well after weaning. Occasional cases of septicemia are also attributable to E. *coli.* 9 different E. *coli* strains where tested. ATCC 11775 (Serovar O1:K1:H7) is used as type strain and was isolated from urine. CECT 501 is an ETEC strain with K91 and K88a,c O:149, H:10. Three *E*. *coli* field isolates from swine were obtained from RIPAC-LABOR GmbH, Potsdam-Golm, Germany. The E. *coli* strains from Ripac are the following: D14_0322-2-1-3 as F4(K88), D13_0981-1-1-2 as F5(K99) and D14_1510-2-1-1 as F6(987p). Four E. *coli* isolates were obtained from the Penn state E. *coli* reference center.

**Table 3: List of E. coli strains as obtained from the Penn state E. coli reference center**

| | **Virulence** | **O-Type** | **H-Type** |
|---|---|---|---|
| E. coli 8.0594 | LT/Stb/F18+ | 141 | 5 |
| E. coli 3.2475 | STa/STb/F18+ | 157 | 19 |
| E. coli 0.2617 | F18+ | 139 | 1 |
| E. coli 2.0419 | LT/K88/F18+ | 180 | 10 |

Bacillus strains were grown in LB-Kelly media (i.e. in media containing 40 g/L soya peptone, 40 g/L dextrin, 1.8 g/L KH₂PO₄, 4.5 g/L K₂HPO₄, 0.3 g/L MgSO₄*7H₂O, 0.2 ml/L Kelly trace metal solution with 3.6 g/L CuSO₄*5H₂O, pH 6) for 16 h at 37°C and 200 rpm in 100 mL shaking flasks. The pathogenic strains were grown under suitable conditions as liquid culture to an optical density (OD 600) of at least 1, then 100 µl were spread with sterile spatula on the surface of agar plates. The pathogens are spread on TSBYE agar plates. Three 9 mm diameter wells were cut into the dried plates. 1st well was used as non-inoculated media control without culture, 2nd well was inoculated with 100 µl not-inhibiting Bacillus strain (*B. cereus* var. toyoi, NCIMB 40112), the 3rd well was inoculated with 100 µl of Bacillus culture. After 24 h incubation under suitable conditions at 37°C, the zone of clearance in mm was determined measuring from the edge of the cut well to the border of the cleared lawn. If there was no halo visible after 24 h of incubation, the incubation of the agar plates was prolonged to 48 h. Each colony was measured twice (horizontally, vertically), then averaged. The results can be found in the following tables.

**Table 4: Inhibitory activity of the strains DSM 33855, DSM 33856, DSM 33857 and DSM 33858 against pathogens of the genera Clostridium, Salmonella, Streptococcus and Staphylococcus**

| Strain | | Salmonella enterica subsp. enterica | | | | | |
|---|---|---|---|---|---|---|---|
| | C. perfringens ATCC 13124 | S. enteritidis DSM 17420 | S. cholera suis DSM 19207 | S. pullorum ATCC 10398 | Streptococcus suis DSM 9682 | Enterococcus cecorum DSM 20638 | Vibrio parahaemolyticus DSM 10027 |
| DSM 33855 | 8.9 | 0 | 0 | 5.5 | 15.3 | 14.2 | 8.2 |
| DSM 33858 | 11.0 | 0 | 0 | 0 | 15.3 | 13.5 | 0 |
| DSM 33856 | 8.9 | 0 | 0 | 9.5 | 14.1 | 9.7 | 6.6 |
| DSM 33857 | 10.0 | 9.3 | 5.4 | 11.3 | 8.8 | 6.9 | 0 |

As can be seen, all four strains are able to inhibit the growth of *C*. *perfringens, S*. *suis* and *Enterococcus cecorum,* while only the strains DSM 33855, DSM 33856 and DSM 33857 are able to inhibit the growth of *Salmonella enterica* subsp. enterica and only the strains DSM 33855 and DSM 33856 are able to inhibit the growth of *Vibrio parahaemolyticus.*

**Table 5: Inhibitory activity of the strains DSM 33855, DSM 33856, DSM 33857 and DSM 33858 against specific pathogenic E. coli strains**

| Strain | E. coli | | | | |
|---|---|---|---|---|---|
| | DSM 30083 = ATCC 11775 = CECT 515 | CECT 501 | D14_0322-2-1-3 | D13_0981-1-1-2 | D14_1510-2-1-1 |
| DSM 33855 | 0 | 0 | 0 | 0 | 0 |
| DSM 33858 | 0 | 0 | 0 | 0 | 0 |
| DSM 33856 | 7.6 | 5.1 | 0 | 5.5 | 0 |
| DSM 33857 | 10.1 | 10.2 | 9.7 | 9.5 | 6.9 |

**Table 6: Inhibitory activity of the strains DSM 33855, DSM 33856, DSM 33857 and DSM 33858 against further specific pathogenic E. coli strains**

| Strain | E. coli | | | |
|---|---|---|---|---|
| | 8.0594 | 3.2475 | 0.2617 | 2.0419 |
| DSM 33855 | 0 | 3.6 | 2.7 | 2.7 |
| DSM 33858 | 0 | 0 | 0 | 0 |
| DSM 33856 | 0 | 6.4 | 6.4 | 6.4 |
| DSM 33857 | 12.2 | 12.6 | 9.2 | 9.3 |

As can be seen the strains DSM 33855, DSM 33856 and DSM 33857 are able to inhibit the growth of pathogenic E. *coli* strains.

### Example 10: Comparative strain performance - quantitative assessment of bile tolerance.

In order to assess the competitiveness of the *Bacillus* strains DSM 33855, DSM 33856, DSM 33857 and DSM 33858, the growth behavior in presence of bile was determined. Readiness of strains to perform in the proximal small intestine in presence of bile at neutral pH right after gastric passage (Argenzio 2004b; Trampel and Duke 2004) was determined by strain growth in VIB media with addition 0.3 wt.-% porcine bile. Overnight culture with 50 µl candidate strain cell suspension and 10 mL VIB in 100 mL conical flask was incubated at 37 °C and 200 rpm, then approximately 50 µl of overnight culture was transferred to 100 well honeycomb plates (Oy Growth Curves Ab Ltd, former Thermo Labsystems, Helsinky, Finland) with 1 mL VIB at pH 7 with 0.3 wt.-% porcine bile in order to obtain OD 0.2 per mL. Strain specific growth at 37 °C and 200 rpm was observed for 48 h with OD determined every 15 min using Bioscreeen C MBR with BioLink software package (Oy Growth Curves Ab Ltd). Averaged triplicate blank OD read of broth with bile only (blanks) were subtracted per culture at each time point before area under the curve (AUC) was calculated. Quantitative assessment for each strain was compared as area under the curve between 0-5 h (AUC5, in OD × time in h), area under the curve between 0-10 h (AUC10 in OD × time in h). Results can be found in Table 7.

**Table 7: Growth of Bacillus strains DSM 33855, DSM 33856, DSM 33857 and DSM 33858 in VIB medium without bile and in presence of 0.3 wt.-% porcine bile.**

| Strain ID | AUC5 | AUC10 |
|---|---|---|
| DSM 33855 | 2.951 | 8.650 |
| DSM 33855 (bile) | 1.203 | 5.434 |
| DSM 33856 | 2.651 | 8.389 |
| DSM 33856 (bile) | 0.760 | 4.722 |
| DSM 33857 | 2.766 | 8.463 |
| DSM 33857 (bile) | 1.582 | 7.587 |
| DSM 33858 | 3.049 | 8.705 |
| DSM 33838 (bile) | 1.233 | 5.298 |

AUC5, area under the curve between time point 0 and 5 h in optical density x h; AUC10, area under the curve between time point 0 and 10 h in optical density x h.

As can be seen, all four strains are not only able to grow in presence of bile, but show quite good growth performance in presence of bile, with strain DSM 33857 showing the best growth performance in presence of bile.

### References:

Argenzio, R. A. (2004b). Digestive and Absorptive Functions of the Intestines, p. 419 - 437. In: Reece, W. O. (ed.), Duke's Physiology of Domestic Animals; Twelfth Edition, Chapter 26; Cornell University Press, Ithaca, New York, USA.

Trampel, D. W. and Duke, G. E. (2004). Avian Digestion, p. 488 - 500. In: Reece, W. O. (ed.), Duke's Physiology of Domestic Animals; Twelfth Edition, Chapter 29; Cornell University Press, Ithaca, New York, USA.

### Example 11: Comparative strain performance relative to state of the art direct-fed microbial (DFM) / probiotic for animal nutrition - quantitative assessment of enzymatic activity.

The strains DSM 33855, DSM 33856, DSM 33857 and DSM 33858 were compared with the state of the art probiotic strain DSM 17299 evaluating the respective carbohydrate degradation and proteolytic activity. Cellulase and xylanase activity were determined as described in Larsen *et al.* (2014). Protease activity was determined in accordance with the Enzymatic Assay of Protease Impurity using Fluorescein Isothiocyanate-Casein as provided by SIGMA (revised version of 08/08/95; Sigma-Aldrich, USA). Analysis was performed in three independent runs, respectively, then averaged as milliunits per microliter solution. Results can be found in Table 8.

**Table 8. Cellulase, xylanase and protease activity of strains DSM 33855, DSM 33856, DSM 33857 and DSM 33858 in comparison to benchmark strain DSM 17299.**

| Strain ID | Cellulase activity (mU/mL) | Xylanase activity (mU/mL) | Protease activity (mU/mL) |
|---|---|---|---|
| DSM 33855 | 134.8 | 14.4 | 11.6 |
| DSM 33856 | 586.9 | 27.7 | 11.6 |
| DSM 33857 | 1559.5 | 70.8 | 263.1 |
| DSM 33858 | 607.5 | 15.2 | 31.4 |
| DSM 17299 | 41.1 | 9.6 | 7.1 |

In direct comparison, all fours strains demonstrated significant higher cellulase, xylanase and protease activity comparing to benchmark strain DSM 17299. In particular the strain DSM 33857 exhibited very high enzymatic activities.

### Reference:

Larsen, N., Thorsen, L., Kpikpi, E. N., Stuer-Lauridsen, B., Cantor, M. D., Nielsen, B., Brockmann, E., Derkx, E. M. F. and Jespersen, L. (2014). Characterization of Bacillus spp. strains for use as probiotic additives in pig feed. Applied microbiology and biotechnology, 98(3), 1105-1118.

### Example 11: Spore stability tests

The endospores of the strains DSM 33855 and DSM 33857 were subjected to various stressors including heat treatment, pH challenge and exposure to high salt (NaCl) concentrations. The experiments were carried out as follows: The strains were grown over night at 37°C and 750 rpm in 50 ml of a medium which induces the sporulation of the cells. The fermentation broth thus obtained was then heated for 10 minutes to a temperature of 80°C. After heat treatment, 3 µl of diluted samples of the fermentation broth were plated on Petri dishes which comprised different kinds of media with challenging conditions. As basis for the Petri dishes served VIB (veal infusion broth) medium. To check salt resistance Petri dishes with VIB medium (pH 7) and either 5 wt.-% or 10 wt.-% of NaCl was prepared. To check pH stability Petri dishes with VIB medium and pH values of 5 and 6 were prepared. Furthermore, Petri dishes with VIB medium (pH 6) were prepared which either comprised 0.4 g/L propionic acid or 0.4 g/L formic acid. After plating of the cells on the Petri dishes, incubation at 37°C took place over night to examine, whether the spores survive such challenging conditions.

It turned out that the spores of the strain DSM 33855 survived all of such challenging conditions, while the strain DSM 33857 survived all such challenging conditions with exception of exposure to 10 wt.-% of NaCl.

### Example 12: Growth under anaerobic conditions

To examine the ability of the cells to grow under anaerobic conditions, the cells were first grown in 10 mL Caso/Yeast media at 37°C for 16 hours (200 rpm) in 100 mL shaking flasks to prepare a pre-culture. After that, 10 mL Caso/Yeast media in Falcon tubes containing 5 g/L sucrose and 5 mM potassium nitrate were inoculated with the respective pre-cultures to adjust an OD of 0.2. After closing the Falcon tubes to inhibit access of air, the tubes were incubated at 37°C for 48 hours without shaking. As next step, the resulting OD(600) was detected as well as the amount of residual sucrose as still contained in the media. Further, also the amount of lactate as contained in the resulting media was determined. As negative control a medium without inoculation was used.

**Table 9. Data regarding the resulting media after inoculation of the strains DSM 33855, DSM 33856, DSM 33857 and DSM 33858 under anaerobic conditions.**

| Strain ID | OD (600) | pH value | Consumed sucrose [g/L] | DL-lactate [g/L] |
|---|---|---|---|---|
| DSM 33855 | 1.40 | 5.5 | 4.12 | 2.71 |
| DSM 33856 | 0.38 | 5.5 | 3.94 | 2.76 |
| DSM 33857 | 0.38 | 6.0 | 3.25 | 0.90 |
| DSM 33858 | 1.01 | 5.5 | 3.47 | 3.87 |
| Test medium | 0 | 7.0 | 0 | 0 |

## Claims

1. Microorganisms which display viral decoy receptors on the surface and preparations thereof.

2. Microorganisms according to claim 1, wherein the microorganisms are endospore-forming microorganisms, preferably of the genus *Bacillus,* in particular selected from the species *B*. *subtilis, B. licheniformis, B. paralicheniformis, B. amyloliquefaciens, B. velezensis, B. pumilus, B. megaterium* and *B*. *coagulans,* more preferably of the species *B*. *subtilis* and *B*. *velezensis.*

3. Microorganisms according to claim 1 or 2, wherein the microorganisms are not genetically modified, preferably naturally occurring microorganisms or spontaneous mutants thereof.

4. Microorganisms according to any of the preceding claims, wherein the microorganisms are able to inhibit the growth of at least one pathogenic bacterium, preferably the growth of at least one, in particular at least two, more preferably of all, of the pathogenic bacteria *Clostridium perfringens*, *Streptococcus suis* and *Enterococcus cecorum.*

5. Microorganisms according to any of the preceding claims, wherein the microorganisms are selected from the Bacillus strains DSM 33855, DSM 33856, DSM 33857 and DSM 33858 and variants thereof and combinations thereof.

6. Microorganisms according to any of the preceding claims, wherein the viral decoy receptor is a compound comprising at least one sugar unit, preferably from one to 10 sugar units, wherein the at least one sugar unit is preferably a sugar alcohol, preferably lactose, a sugar acid, preferably sialic acid (Sia), or a sugar amine, preferably selected from N-acetylgalactosamine, N-acetylglucosamine and N-acetylmannosamine.

7. Microorganisms according to any of the preceding claims, wherein the viral decoy receptor is or comprises a glycan, preferably selected from sialic acid (Sia) and Sia containing glycans, in particular selected from NeuGc alpha 2,3 Sialic acids, Neu5,9Ac2 Sialic acids, α2,3 N-linked and O-linked sialic acids, NeuAc alpha 2,6 Sialic acids, alpha 2,3 Sialic acids, alpha 2,6 Sialic acids, 9-O-acetylated Sialic acids, Neu5Gc Sialic acids, Neu5Ac Sialic acids and linear sialylated pentasaccharides, glycosaminoglycans, mucin-core-2, LNT (lacto-N-tetraose), LNnT (lacto-N-neotetraose), heparan sulfate, chondroitin sulfate and A/O Histo-blood group antigen (HBGA).

8. Microorganisms according to any of the preceding claims, wherein the viral decoy receptor binds to at least one virolectin, preferably selected from coronaviral and rotaviral virolectins, in particular selected from PEDV-S1 and variants of rotavirus P8*, in particular selected from P6_Z84_VP8* and P19_VP8*.

9. Microorganisms according to any of the preceding claims, wherein the microorganisms are able to bind viruses, preferably at least two or three viruses, in particular to Sia targeting viruses, preferably selected from the families Myxoviridae, Coronaviridae, Reoviridae, Noroviridae, Picornaviridae, Adenoviridae, Anelloviridae, Asfarviridae, Baculoviridae, Circoviridae, Geminiviridae, Hepadnaviridae, Iridoviridae, Nanoviridae, Nimaviridae, Nudiviridae, Papillomaviridae, Parvoviridae, Polyomaviridae, Flaviviridae, Bunyaviridae, Filoviridae, Arenaviridae and Poxviridae, more preferably of the families Coronaviridae or Reoviridae.

10. Microorganisms according to any of the preceding claims, wherein the microorganisms produce and/or display at least two different kinds, preferably at least three or four different kinds, of viral decoy receptors, in particular selected from sialic acid (Sia) and Sia containing glycans, preferably selected from NeuGc alpha 2,3 Sialic acids, Neu5,9Ac2 Sialic acids, α2,3 N-linked and O-linked sialic acids, NeuAc alpha 2,6 Sialic acids, alpha 2,3 Sialic acids, alpha 2,6 Sialic acids, 9-O-acetylated Sialic acids, Neu5Gc Sialic acids, Neu5Ac Sialic acids and linear sialylated pentasaccharides, glycosaminoglycans, mucin-core-2, LNT (lacto-N-tetraose), LNnT (lacto-N-neotetraose), heparan sulfate, chondroitin sulfate and A/O Histo-blood group antigen (HBGA), wherein preferably at least one of the viral decoy receptors is a Sia containing glycan and at least one further of the viral decoy receptors is a non-Sia containing glycan.

11. Microorganisms according to any of the preceding claims, wherein the microorganisms possess at least one, preferably at least two or three, more preferably at least five or six, further characteristics selected from: ability to grow fast in large-scale bioreactors; ability to multiply and produce viral decoy receptors *in situ*; ability to grow in the presence of bile; ability to form endospores; ability to produce at least one enzyme, preferably selected from cellulase, xylanase and protease; sensitivity with respect to at least 8, 10 or 12 different antibiotics; ability to grow under anaerobic conditions and preferably to produce lactic acid under anaerobic conditions; ability to inhibit the growth of pathogenic bacteria selected from *E. coli*, *Vibrio parahaemolyticus* and *Staphylococcus aureus* subsp. aureus.

12. Preparations of microorganisms of any of the preceding claims, wherein the preparations contain an effective mixture of components as contained in and/or produced by such microorganisms, wherein the preparations are preferably selected from optionally dried fermentation broths of such microorganisms and optionally dried supernatants of such fermentation broths.

13. Compositions containing at least one microorganism according to any of claims 1 to 11 and/or at least one preparation according to claim 12, wherein the compositions are selected from feed and food compositions, feed and food additives, water and aqueous suspensions, compositions for treating plants, decontaminant and sanitizing compositions, wherein the feed and food compositions preferably contain at least one, more preferably at least two or three, further ingredients selected from carriers, proteins, carbohydrates, fats, further probiotics, prebiotics, enzymes, vitamins, immune modulators, milk replacers, minerals, amino acids, carriers, coccidiostats, acid-based products and/or medicines, such as antibiotics.

14. Pharmaceutical composition containing at least one microorganism according to any of claims 1 to 11 and/or at least one preparation according to claim 12 and at least one pharmaceutically acceptable carrier, in particular for treating and/or preventing and/or mitigating the course of a disease, wherein the disease is preferably a viral and/or bacterial disease, in particular selected from diarrhea, necrotic enteritis and influenza.

15. Method of feeding animals, wherein the animals are fed with at least one microorganism according to any of claims 1 to 11 and/or with at least one preparation according to claim 12 and/or with a food or feed composition according to claim 13 and/or with a pharmaceutical composition according to claim 14, wherein the animals are preferably selected from mammals, in particular swine and ruminants, birds, in particular chickens, and aquatic animals.

16. Method of treating and/or preventing and/or mitigating the course of a disease, preferably a viral and/or bacterial disease, wherein at least one microorganism according to any of claims 1 to 11 and/or at least one preparation according to claim 12 and/or a food or feed composition according to claim 13 and/or a pharmaceutical composition according to claim 14 is administered to animals in need thereof.

17. Use of at least one microorganism according to any of claims 1 to 11 and/or at least one preparation according to claim 12 for the manufacture of a feedstuff and/or a foodstuff and/or a pharmaceutical composition, in particular a pharmaceutical composition for the treatment or prevention or mitigation of the course of a disease, in particular a viral and/or bacterial disease, wherein the viral and/or bacterial disease is preferably selected from diarrhea, necrotic enteritis and influenza.

18. Method of identifying microorganism which produce or display viral decoy receptors, comprising the following steps:
a) Heterologous expression and purification of viral receptor binding proteins, in particular virolectins, and their assembly on a carrier;
b) Exposing the carriers carrying the viral receptor binding proteins to a collection of microorganisms;
c) Examining whether microorganisms are bound to the viral particles on the carrier by centrifugation of the microorganisms and examination of the residual, unbound, nanoparticles in the supernatant.
